# EUROPEAN PATENT APPLICATION

(11) **EP 4 674 962 A1**
(43) Date of publication of application: **07.01.2026**
(21) Application number: 24763895.0
(22) Date of filing: 27.02.2024
(51) Int. Cl.: C12N 15/11, A61K 31/7088, A61K 31/712, A61K 31/7125, A61P 25/00, C12N 15/113

(54) **OLIGONUCLEOTIDE, AND REST EXPRESSION SUPPRESSING AGENT AND PHARMACEUTICAL COMPOSITION USING SAME**

(30) Priority: 28.02.2023 JP 2023029297
(71) Applicant: The University of Osaka, Osaka 565-0871 (JP)
(72) Inventor: SHIMOJO, Masahito, Osaka 565-0871 (JP); OBIKA, Satoshi, Osaka 565-0871 (JP); TAMENORI, Yusuke, Osaka 565-0871 (JP)
(74) Representative: Schlich
(86) International application number: PCT/JP2024/006991
(87) International publication number: WO 2024/181416

(57) **Abstract**

An oligonucleotide of the present invention includes a nucleotide sequence complementary to a continuous sequence with at least 15 bases in a target region included in a base sequence of SEQ ID NO: 1, and can regulate the expression of REST. The oligonucleotide and pharmacologically acceptable salt thereof of the present invention is applicable as, for example, a material to be included in therapeutic agents for various nervous system diseases (e.g., malignant tumors, dementia, and central nervous system diseases), diabetes, and the like, which are caused by the expression of the REST.

## Description

### Technical Field

The present invention relates to an oligonucleotide, and a REST expression suppressing agent and a pharmaceutical composition using the same.

### Background Art

In neurological diseases caused by genetic factors or aging, the expression of many neuron-specific genes is suppressed. A transcription repressor REST (RE1-silencing transcription factor), which is associated with the expression of neuron-specific genes, suppresses the expression of a neuron-specific gene by binding to the 21-bp RE1 element on the gene.

The REST is not expressed in most of the normal central nervous systems as a result of pre-mRNA splicing, but is abnormally expressed in neuroblastoma (Non-Patent Documents 1 to 3) and malignant brain tumors such as medulloblastoma (Non-Patent Document 4) and glioblastoma (Non-Patent Documents 5 to 8) and functions as a carcinogenic factor (Non-Patent Document 9). It is also reported that the expression of the REST is gradually increased with aging, which results in suppression of the expression of neural genes, thereby causing dementia (Non-Patent Document 10).

Furthermore, it is reported that the expression of the REST causes fibrositis. Fibrositis is a disease that develops with a high incidence in Japan as in the West, and about 1.7% (about two millions) of the Japanese develops it. It is reported that promoted expression of the REST is associated with fibrositis and neuropathic pain (Non-Patent Document 11).

Accordingly, there is demand for development of a therapeutic agent that can target and regulate the abnormal expression of the REST, which has an influence on various neurological diseases.

### Related Art Documents

### Non-Patent Documents

[Non-Patent Document 1] Neuron-specific splicing of zinc finger transcription factor REST/NRSF/XBR is frequent in neuroblastomas and conserved in human, mouse and rat. Brain Res Mol Brain Res. 1999; 72:30.
[Non-Patent Document 2] The REST gene signature predicts drug sensitivity in neuroblastoma cell lines and is significantly associated with neuroblastoma tumor stage. Int J Mol Sci. 2014; 15:11220.
[Non-Patent Document 3] The risk-associated long noncoding RNA NBAT-1 controls neuroblastoma progression by regulating cell proliferation and neuronal differentiation. Cancer Cell. 2014; 26:722.
[Non-Patent Document 4] The neuronal repressor REST/NRSF is an essential regulator in medulloblastoma cells. Nat Med. 2000; 6:826.
[Non-Patent Document 5] Neuronal expression of zinc finger transcription factor REST/NRSF/XBR gene. J Neurosci. 1998; 18:1280.
[Non-Patent Document 6] REST controls self-renewal and tumorigenic competence of human glioblastoma cells. PLoS ONE. 2012; 7:e38486.
[Non-Patent Document 7] A REST derived gene signature stratifies glioblastomas into chemotherapy resistant and responsive disease. BMC Genomics. 2012; 13:686.
[Non-Patent Document 8] Inhibition of REST suppresses proliferation and migration in glioblastoma cells. Int J Mol Sci. 2016; 17:664.
[Non-Patent Document 9] Ubiquitination and deubiquitination of REST and its roles in cancers. FEBS Lett. 2012; 586:1602.
[Non-Patent Document 10] REST and stress resistance in aging and Alzheimer' s disease. Nature. 2014; 507(7493): 448.
[Non-Patent Document 11] Increased NRSF/REST in anterior cingulate cortex contributes to diabetes-related neuropathic pain. Biochem Biophys Res Commun. 2020; 527:785.

### Summary of Invention

### Problem to be Solved by the Invention

The present invention is to solve the aforementioned problems, and it is an object thereof to provide an oligonucleotide that can target and regulate the abnormal expression of the REST, which has an influence on various neurological diseases, and a REST expression suppressing agent and a pharmaceutical composition using the same.

### Means for Solving the Problem

The present invention provides an oligonucleotide or pharmacologically acceptable salt thereof capable of regulating expression of REST, comprising a nucleotide sequence complementary to a continuous sequence with at least 15 bases in a target region included in a base sequence of SEQ ID NO: 1.

In one embodiment, the oligonucleotide has 16 to 25 bases.

In one embodiment, the oligonucleotide includes a sequence complementary to a target region having a sequence within a base sequence between positions 900 to 4700 of SEQ ID NO: 1.

In one embodiment, a 5' terminus of the target region corresponds to position 923, 1522, 1523, 1524, 2248, 4082, 4083, 4084, 4085, 4629, 4631, 4632, 4633, 4634, 4635, or 4645 of SEQ ID NO: 1.

In one embodiment, the oligonucleotide is
REST-923-17(L) (SEQ ID NO: 18),
REST-1522-17(L) (SEQ ID NO: 19),
REST-1523-17(L) (SEQ ID NO: 20),
REST-1524-17(L) (SEQ ID NO: 21),
REST-2248-17(L) (SEQ ID NO: 22),
REST-4082-17(L) (SEQ ID NO: 23),
REST-4083-17(L) (SEQ ID NO: 24),
REST-4084-17(L) (SEQ ID NO: 25),
REST-4085-17(L) (SEQ ID NO: 26),
REST-4629-17(L) (SEQ ID NO: 27),
REST-4631-17(L) (SEQ ID NO: 28),
REST-4632-17(L) (SEQ ID NO: 29),
REST-4633-17(L) (SEQ ID NO: 30),
REST-4634-17(L) (SEQ ID NO: 31),
REST-4635-17(L) (SEQ ID NO: 32),
REST-4645-17(L) (SEQ ID NO: 33),
REST-2248-17(Y) (SEQ ID NO: 34),
REST-4082-17(Y) (SEQ ID NO: 35), or
REST-4633-17(Y) (SEQ ID NO: 36).

In one embodiment, the oligonucleotide or pharmacologically acceptable salt thereof comprises at least one nucleoside structure represented by Formula (I) below:

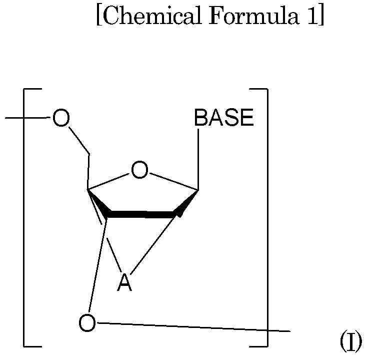

### (in Formula (I),

BASE represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms, and
A is a divalent group represented by: 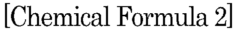 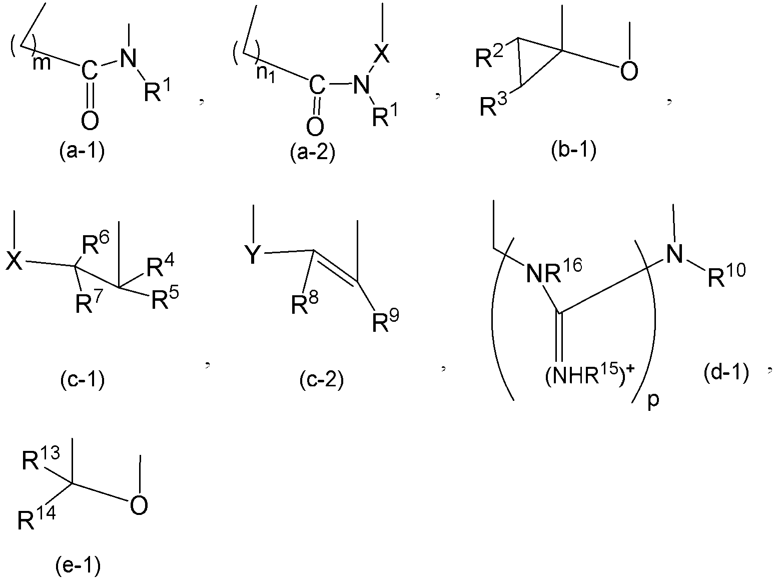 or
where
R¹ represents a hydrogen atom, an alkyl group having 1 to 7 carbon atoms that may be branched or cyclic, an alkenyl group having 2 to 7 carbon atoms that may be branched or cyclic, an aryl group having 3 to 12 carbon atoms that may have any one or more substituents selected from the α group and may have a hetero atom, an aralkyl group with an aryl moiety having 3 to 12 carbon atoms that may have any one or more substituents selected from the α group and may have a hetero atom, or an amino group protecting group for nucleic acid synthesis;
R² and R³ are independently a hydrogen atom, an alkyl group having 1 to 7 carbon atoms that may have undergone substitution with an aryl group having 3 to 12 carbon atoms that may have a hetero atom, and may be branched or cyclic, or an aralkyl group with an aryl moiety having 3 to 12 carbon atoms that may have a hetero atom, or
R² and R³ together represent -(CH₂)_{q}- (where q is an integer from 2 to 5);
R⁴ and R⁵ are independently a group selected from the group consisting of a hydrogen atom, a hydroxy group, alkyl groups having 1 to 7 carbon atoms that may be branched or cyclic, alkoxy groups having 1 to 7 carbon atoms that may be branched or cyclic, an amino group, and an amino group protected by a protecting group for nucleic acid synthesis, or R⁴ and R⁵ together form =C(R¹¹)R¹² (where R¹¹ and R¹² independently represent a hydrogen atom, a hydroxy group, a hydroxy group protected by a protecting group for nucleic acid synthesis, a mercapto group, a mercapto group protected by a protecting group for nucleic acid synthesis, an amino group, a linear or branched alkoxy group having 1 to 6 carbon atoms, a linear or branched alkylthio group having 1 to 6 carbon atoms, a cyanoalkoxy group having 1 to 6 carbon atoms, or a linear or branched alkylamino group having 1 to 6 carbon atoms);
R⁶ and R⁷ are independently a hydrogen atom, an alkyl group having 1 to 7 carbon atoms that may be branched or cyclic, an alkoxy group having 1 to 7 carbon atoms that may be branched or cyclic, or a linear or branched alkylthio group having 1 to 6 carbon atoms;
R⁸ represents a hydrogen atom, an alkyl group having 1 to 7 carbon atoms that may be branched or cyclic, an alkoxy group having 1 to 7 carbon atoms that may be branched or cyclic, or a linear or branched alkylthio group having 1 to 6 carbon atoms;
R⁹ is a hydrogen atom, a hydroxy group, an alkyl group having 1 to 7 carbon atoms that may be branched or cyclic, an alkoxy group having 1 to 7 carbon atoms that may be branched or cyclic, an amino group, or an amino group protected by a protecting group for nucleic acid synthesis;
R¹⁰ is a hydrogen atom, an alkyl group having 1 to 7 carbon atoms that may be branched or cyclic, an amino group protecting group, or 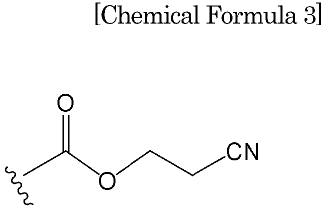
or -(C=(NHR¹⁷)⁺)-NR¹⁸R¹⁹ (where R¹⁷, R¹⁸, and R¹⁹ are independently a hydrogen atom, an alkyl group having 1 to 7 carbon atoms that may be branched or cyclic, an amino group protecting group, or 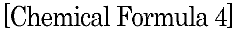 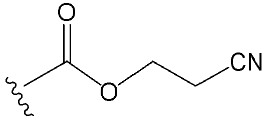 );
R¹³ and R¹⁴ are independently a group selected from the group consisting of a hydrogen atom, a hydroxy group, alkyl groups having 1 to 7 carbon atoms that may be branched or cyclic, alkoxy groups having 1 to 7 carbon atoms that may be branched or cyclic, an amino group, and an amino group protected by a protecting group for nucleic acid synthesis;
m is an integer from 0 to 2;
n is an integer of 0 or 1;
when R¹⁰ is a hydrogen atom, an alkyl group having 1 to 7 carbon atoms that may be branched or cyclic, an amino group protecting group, or 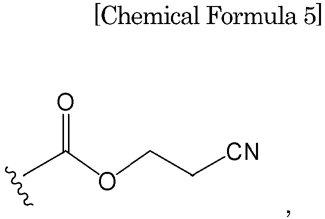
p is 1, and R¹⁵ and R¹⁶ are independently a hydrogen atom, an alkyl group having 1 to 7 carbon atoms that may be branched or cyclic, an amino group protecting group, or 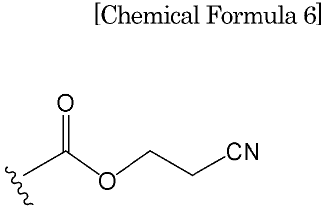 or
when R¹⁰ is -(C=(NHR¹⁷)⁺)-NR¹⁸R¹⁹, p is 0;
X is an oxygen atom, a sulfur atom, or an amino group; and
Y is an oxygen atom or a sulfur atom).

In a further embodiment, the oligonucleotide is a gapmer that includes a gap region having 9 to 15 bases, a 5' wing having 3 to 5 bases, and 3' wing having 3 to 5 bases,
the gap region is located between the 5' wing and the 3' wing, and
the 5' wing and the 3' wing include at least one nucleoside structure represented by Formula (I).

In one embodiment, bonds between nucleotides included in the oligonucleotide include phosphorothioate.

The present invention also provides a REST expression suppressing agent comprising the above oligonucleotide or pharmacologically acceptable salt thereof.

The present invention also provides a pharmaceutical composition comprising the above oligonucleotide or pharmacologically acceptable salt thereof.

In one embodiment, the pharmaceutical composition of the present invention is used for treating a nervous system disease.

### Effects of the Invention

With the present invention, it is possible to target the REST and to, for example, effectively suppress the expression of the REST. Accordingly, the oligonucleotide and pharmacologically acceptable salt thereof of the present invention are also useful for, for example, development of therapeutic agents for various nervous system diseases (e.g., malignant tumors, dementia, and central nervous system diseases), diabetes, and the like, which are caused by the expression of the REST.

### Brief Description of Drawings

FIG. 1 is a graph illustrating the results of analyses of the REST expression levels conducted in Example 3 using gapmer-type antisense oligonucleotides (ASOs).
FIG. 2(a) is a graph illustrating the results (REST) of ASO concentration optimization conducted in Example 4 using REST-4632-17(L) and NEG2, and FIG. 2(b) is a graph illustrating the results (Actin) of the ASO concentration optimization.
FIG. 3 is a graph illustrating the results of analyses of the REST expression levels conducted in Example 5 using gapmer-type ASOs.
FIG. 4 is a graph illustrating the results of ASO concentration-dependent activity conducted in Example 6 using REST-2248-17(L), REST-4082-17(L), and REST-4633-17(L)
FIG. 5 is a graph illustrating relative REST expression levels versus the logarithmic values of the ASO concentrations, made based on the results of ASO concentration-dependent activity conducted in Example 6 using REST-2248-17(L), REST-4082-17(L), and REST-4633-17(L).
FIG. 6 is a graph illustrating the results of analyses of suppression of cell proliferation conducted on A549 cells in Example 7 using REST-2248-17(L), REST-4082-17(L), and REST-4633-17(L).
FIG. 7 is a graph illustrating the results of analyses of suppression of cell proliferation conducted on U-251 cells in Example 8 using REST-2248-17(L), REST-4082-17(L), and REST-4633-17(L).
FIG. 8 is a graph illustrating the results of cell proliferation ratios conducted on A549 cells in Example 9 using REST-2248-17(L), REST-4082-17(L), and REST-4633-17(L).
FIG. 9 is a graph illustrating the results of analyses of cell proliferation ratios conducted on U-251 cells in Example 10 using REST-2248-17(L), REST-4082-17(L), and REST-4633-17(L).
FIG. 10 shows photographs illustrating the results of REST protein expression conducted in Example 11 using anti-REST antibodies.
FIG. 11 is a graph illustrating the results of analyses of the REST expression levels conducted in Example 12 using gapmer-type ASOs.

### Description of Embodiments

### Definitions of Terms

The following definitions shall apply throughout the specification.

The term "nucleoside" as used herein encompasses "nucleosides" in which a purine base or a pyrimidine base binds to sugar, as well as those in which a heteroaromatic ring and an aromatic hydrocarbon ring other than purine and pyrimidine, serving as a substitute for a purine base or a pyrimidine base, binds to sugar. A natural nucleoside is also referred to as a "native nucleoside". A modified non-natural nucleoside is also referred to as a "modified nucleoside", and in particular, a nucleotide in which a sugar moiety is modified is referred to as a "sugar-modified nucleoside". The term "nucleotide" means a compound obtained through binding of a phosphate group to sugar of a nucleoside.

The term "oligonucleotide" as used herein refers to a polymer of "nucleotides" in which the same or different "nucleosides" are bound via phosphodiester bonds or other bonds, and encompasses natural oligonucleotides and non-natural oligonucleotides. Preferable examples of the non-natural "oligonucleotides" include sugar derivatives with sugar moieties being modified; thioated derivatives with phosphate diester moieties being thioated; esters with terminal phosphate moieties being esterificated; and amides with amino groups on purine bases being amidated. The sugar derivatives with sugar moieties being modified are more favorable.

The term "pharmacologically acceptable salt thereof" as used herein refers to a physiologically and pharmaceutically acceptable salt of the oligonucleotide according to the present invention that include, for example, at least one nucleoside structure represented by Formula (I) below, that is, a salt that keeps desired biological activity of that oligonucleotide and does not exhibit undesired toxic effects.

Examples of such salts include metal salts including alkali metal salts such as sodium salts, potassium salts, and lithium salts, alkaline earth metal salts such as calcium salts and magnesium salts, aluminum salts, iron salts, zinc salts, copper salts, nickel salts, cobalt salts, and the like; amine salts including inorganic salts such as ammonium salts, organic salts such as t-octylamine salts, dibenzylamine salts, morpholine salts, glucosamine salts, phenylglycine alkylester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, tris(hydroxymethyl)aminomethane salts, and the like; inorganic acid salts including halide hydroacid salts such as hydrofluoric acid salts, hydrochloric acid salt, hydrobromic acid salts, and hydroiodic acid salts, nitrates, perchlorates, sulfates, phosphates, and the like; organic acid salts including lower-alkane-sulfonates such as methanesulfonates, trifluoromethanesulfonates, and ethanesulfonates, arylsulfonates such as benzenesulfonates and p-toluenesulfonates, acetates, malates, fumarates, succinates, citrates, tartrates, oxalates, maleates, and the like; and amino acid salts such as glycine salts, lysine salts, arginine salts, ornithine salts, glutamates, and aspartates.

The term "complementary" as used herein means a relationship between nucleic acid bases that can form the so-called Watson-Crick base pair (natural base pairing) or a non-Watson-Crick base pair (e.g., a Hoogsteen base pair, a wobble base pair, or the like) via a hydrogen bond. Therefore, the wording "complementary sequence" implicates not only a sequence that is completely complementary to (i.e., capable of hybridizing, without mismatches, with) a sequence of interest (e.g., a complementary sequence in a complementary strand, a target region in targe RNA, or the like), but also sequences with one to several (e.g., 2, 3, 4, 5, or more) mismatches as long as they can hybridize with a target sequence under stringent conditions or under physiological conditions for mammalian cells. That is to say, it is allowable that the complementary strand includes mismatches. Examples of such sequences include those having 80% or more (e.g., 85% or more, 90% or more, 95% or more, 96% or more, 97% or more, 98% or more, or 99% or more) identity to a sequence that is completely complementary to a target sequence, and a sequence having 100% identity thereto is most preferable.

The term "alkyl group having 1 to 3 carbon atoms" as used herein encompasses any alkyl groups having 1 to 3 carbon atoms. Specific examples thereof are a methyl group, an ethyl group, an n-propyl group, and an isopropyl group.

The term "linear alkyl group having 1 to 6 carbon atoms" as used herein encompasses any linear alkyl groups having 1 to 6 carbon atoms. Specific examples thereof are a methyl group, an ethyl group, an n-propyl group, an n-butyl group, an n-pentyl group, and an n-hexyl group.

The term "linear alkoxy group having 1 to 6 carbon atoms" as used herein encompasses alkoxy groups that include any linear alkyl group having 1 to 6 carbon atoms. Examples thereof include a methyloxy group, an ethyloxy group, an n-propyloxy group, and the like. The term "linear or branched alkoxy group having 1 to 6 carbon atoms" as used herein encompasses alkoxy groups that include any linear or branched alkyl group having 1 to 6 carbon atoms. Examples thereof include a methyloxy group, an ethyloxy group, an n-propyloxy group, an isopropyloxy group, an n-butyloxy group, an isobutyloxy group, a tert-butyloxy group, an n-pentyloxy group, an isopentyloxy group, and the like.

The term "linear alkylthio group having 1 to 6 carbon atoms" as used herein encompasses alkylthio groups that include any linear alkyl group having 1 to 6 carbon atoms. Examples thereof include a methythio group, an ethylthio group, an n-propylthio group, and the like. The term "linear or branched alkylthio group having 1 to 6 carbon atoms" as used herein encompasses alkylthio groups that include any linear or branched alkyl group having 1 to 6 carbon atoms. Examples thereof include a methythio group, an ethylthio group, an n-propylthio group, an isopropylthio group, an n-butylthio group, an isobutylthio group, a tert-butylthio group, an n-pentylthio group, an isopentylthio group, and the like.

The term "cyanoalkoxy group having 1 to 6 carbon atoms" as used herein refers to a group obtained by substituting at least one hydrogen atom included in the above-mentioned linear alkoxy group having 1 to 6 carbon atoms with a cyano group.

The term "linear alkylamino group having 1 to 6 carbon atoms" as used herein encompasses groups obtained by substituting one or two hydrogen atoms included in an amino group with a linear alkyl group having 1 to 6 carbon atoms. Examples thereof include a methylamino group, a dimethylamino group, an ethylamino group, a methylethylamino group, and diethylamino group. The term "linear or branched alkylamino group having 1 to 6 carbon atoms" as used herein encompasses groups obtained by substituting one or two hydrogen atoms included in an amino group with any linear or branched alkyl group having 1 to 6 carbon atoms. Examples thereof include a methylamino group, a dimethylamino group, an ethylamino group, a methylethylamino group, a diethylamino group, an n-propylamino group, a di-n-propylamino group, an isopropylamino group, and diisopropylamino group.

The term "alkyl group having 1 to 7 carbon atoms that may be branched or cyclic" as used herein encompasses any linear alkyl groups having 1 to 7 carbon atoms, any branched alkyl groups having 3 to 7 carbon atoms, and any cyclic alkyl groups having 3 to 7 carbon atoms. Such groups may also be referred to merely as "lower alkyl groups". Examples of any linear alkyl groups having 1 to 7 carbon atoms include a methyl group, an ethyl group, an n-propyl group, an n-butyl group, an n-pentyl group, an n-hexyl group, and an n-heptyl group, examples of any branched alkyl groups having 3 to 7 carbon atoms include an isopropyl group, an isobutyl group, a tert-butyl group, an isopentyl group, and the like, and examples of any cyclic alkyl groups having 3 to 7 carbon atoms include a cyclobutyl group, a cyclopentyl group, a cyclohexyl group, and the like.

The term "alkenyl group having 2 to 7 carbon atoms that may be branched or cyclic" as used herein encompasses any linear alkenyl groups having 2 to 7 carbon atoms, any branched alkenyl groups having 3 to 7 carbon atoms, and any cyclic alkenyl groups having 3 to 7 carbon atoms. Such groups may also be referred to merely as "lower alkenyl groups". Examples of any linear alkenyl groups having 2 to 7 carbon atoms include an ethenyl group, a 1-propenyl group, a 2-propenyl group, a 1-butenyl group, a 2-butenyl group, a 1-pentenyl group, a 2-pentenyl group, a 3-pentenyl group, a 4-pentenyl group, 1-hexenyl group, and the like, examples of any branched alkenyl groups having 3 to 7 carbon atoms include an isopropenyl group, a 1-methyl-1-propenyl group, a 1-methyl-2-propenyl group, a 2-methyl-1-propenyl group, a 2-methyl-2-propenyl group, a 1-methyl-2-butenyl group, and the like, and examples of any cyclic alkenyl groups having 3 to 7 carbon atoms include a cyclobutenyl group, a cyclopentenyl group, a cyclohexenyl group, and the like.

The term "alkoxy group having 1 to 7 carbon atoms that may be branched or cyclic" as used herein encompasses any linear alkoxy groups having 1 to 7 carbon atoms, any branched alkoxy groups having 3 to 7 carbon atoms, and any cyclic alkoxy groups having 3 to 7 carbon atoms. Such groups may also be referred to as "lower alkoxy groups". Examples of any linear alkoxy groups having 1 to 7 carbon atoms include a methoxy group, an ethoxy group, an n-propoxy group, an n-butyloxy, an n-pentyloxy group, an n-hexyloxy group, and an n-heptyloxy group, examples of any branched alkoxy groups having 3 to 7 carbon atoms include an isopropoxy group, an isobutyloxy group, a tert-butyloxy group, an isopentyloxy group, and the like, and examples of any cyclic alkoxy groups having 3 to 7 carbon atoms include a cyclobutyloxy group, a cyclopentyloxy group, a cyclohexyloxy group, and the like.

The term "aryl group having 3 to 12 carbon atoms that may include a hetero atom" as used herein encompasses any aryl groups having 6 to 12 carbon atoms that are constituted by only a hydrocarbon, and any heteroaryl groups having 3 to 12 carbon atoms obtained by substituting at least one carbon atom included in the ring structure of the above-mentioned aryl groups with a hetero atom (e.g., a nitrogen atom, an oxygen atom, a sulfur atom, and a combination thereof). Examples of the aryl groups having 6 to 12 carbon atoms include a phenyl group, a naphthyl group, an indenyl group, an azulenyl group, and the like, and examples of any heteroaryl groups having 3 to 12 carbon atoms include a pyridyl group, a pyrrolyl group, a quinolyl group, an indolyl group, an imidazolyl group, a furyl group, a thienyl group, and the like.

Examples of the term "aralkyl group with an aryl moiety having 3 to 12 carbon atoms that may include a heteroatom" as used herein include a benzyl group, a phenethyl group, a naphthylmethyl group, a 3-phenylpropyl group, a 2-phenylpropyl group, a 4-phenylbutyl group, a 2-phenylbutyl group, a pyridylmethyl group, an indolylmethyl group, a furylmethyl group, a thienylmethyl group, a pyrrolylmethyl group, a 2-pyridylethyl group, a 1-pyridylethyl group, a 3-thienylpropyl group, and the like.

Examples of the term "acyl group" as used herein include aliphatic acyl groups and aromatic acyl groups. Specifically, examples of the aliphatic acyl groups include alkylcarbonyl groups such as a formyl group, an acetyl group, a propionyl group, a butyryl group, an isobutyryl group, a pentanoyl group, a pivaloyl group, a valeryl group, an isovaleryl group, an octanoyl group, a nonanoyl group, a decanoyl group, a 3-methylnonanoyl group, a 8-methylnonanoyl group, a 3-ethyloctanoyl group, a 3,7-dimethyloctanoyl group, an undecanoyl group, a dodecanoyl group, a tridecanoyl group, a tetradecanoyl group, a pentadecanoyl group, a hexadecanoyl group, a 1-methylpentadecanoyl group, a 14-methylpentadecanoyl group, a 13,13-dimethyltetradecanoyl group, a heptadecanoyl group, a 15-methylhexadecanoyl group, an octadecanoyl group, a 1-methylheptadecanoyl group, a nonadecanoyl group, an eicosanoyl group, and a heneicosanoyl group; carboxylated alkylcarbonyl groups such as a succinoyl group, a glutaroyl group, and an adipoyl group; halogeno lower-alkyl-carbonyl groups such as a chloroacetyl group, a dichloroacetyl group, a trichloroacetyl group, and a trifluoroacetyl group; lower-alkoxy-lower-alkyl-carbonyl groups such as a methoxyacetyl group; and unsaturated alkylcarbonyl groups such as an (E)-2-methyl-2-butenoyl group. Examples of the aromatic acyl groups include arylcarbonyl groups such as a benzoyl group, an α-naphthoyl group, and a β-naphthoyl group; halogeno arylcarbonyl groups such as a 2-bromobenzoyl group and a 4-chlorobenzoyl group; low-alkylated arylcarbonyl groups such as a 2,4,6-trimethylbenzoyl group and a 4-toluoyl group; low-alkoxylated arylcarbonyl groups such as a 4-anisoyl group; carboxylated arylcarbonyl groups such as a 2-carboxybenzoyl group, a 3-carboxybenzoyl group, and a 4-carboxybenzoyl group; nitrated arylcarbonyl groups such as a 4-nitrobenzoyl group and a 2-nitrobenzoyl group; low-alkoxycarbonylated arylcarbonyl groups such as a 2-(methoxycarbonyl)benzoyl group; arylated arylcarbonyl groups such as a 4-phenylbenzoyl group; and the like. A formyl group, an acetyl group, a propionyl group, a butyryl group, an isobutyryl group, a pentanoyl group, a pivaloyl group, and a benzoyl group are favorable.

Examples of the term "silyl group" as used herein include tri-lower-alkyl-silyl groups such as a trimethylsilyl group, a triethylsilyl group, an isopropyldimethylsilyl group, a t-butyldimethylsilyl group, a methyldiisopropylsilyl group, a methyldi-t-butylsilyl group, and a triisopropylsilyl group; and tri-lower-alkyl-silyl groups that have undergone substitution with one or two aryl groups, such as a diphenylmethylsilyl group, a butyldiphenylbutylsilyl group, a diphenylisopropylsilyl group, and a phenyldiisopropylsilyl group. A trimethylsilyl group, a triethylsilyl group, a triisopropylsilyl group, a t-butyldimethylsilyl group, and a t-butyldiphenylsilyl group are favorable, and a trimethylsilyl group is more favorable.

Examples of the term "halogen atom" as used herein include a fluorine atom, a chlorine atom, a bromine atom, and an iodine atom. A fluorine atom or a chlorine atom is favorable.

Examples of the term "halide ion" as used herein include a fluoride ion, a chloride ion, a bromide ion, and an iodide ion. A fluoride ion or a chloride ion is favorable.

"Protecting groups" in the terms "amino group protecting group for nucleic acid synthesis", "hydroxy group protecting group for nucleic acid synthesis", "hydroxy group protected by a protecting group for nucleic acid synthesis", "phosphate group protected by a protecting group for nucleic acid synthesis", and "mercapto group protected by a protecting group for nucleic acid synthesis" as used herein are not particularly limited as long as they can stably protect an amino group, a hydroxy group, a phosphate group, or a mercapto group during nucleic acid synthesis. Specifically, the protecting groups are stable under an acidic or neutral condition and can be cleaved using chemical techniques such as hydrogenolysis, hydrolysis, electrolysis, and photolysis. Examples of such protecting groups include lower alkyl groups, lower alkenyl groups, acyl groups, tetrahydropyranyl or tetrahydrothiopyranyl groups, tetrahydrofuranyl or tetrahydrothiofuranyl groups, silyl groups, lower-alkoxy-methyl groups, low-alkoxilated lower-alkoxy-methyl groups, halogeno lower-alkoxy-methyl groups, low-alkoxilated ethyl groups, halogenated ethyl groups, methyl groups that have undergone substitution with 1 to 3 aryl groups, "methyl groups that have undergone substitution with 1 to 3 aryl groups in which an aryl ring has undergone substitution with a lower alkyl group, lower alkoxy group, halogen atom, or cyano group", lower-alkoxy-carbonyl groups, "aryl groups that have undergone substitution with a halogen atom, lower alkoxy group, or nitro group", "lower-alkoxy-carbonyl groups that have undergone substitution with a halogen atom or tri-lower-alkyl-silyl group", alkenyloxycarbonyl groups, "aralkyloxycarbonyl groups in which an aryl ring may have undergone substitution with a lower alkoxy group or nitro group", "lower-alkoxy-carbonyl groups that have undergone substitution with a cyano group", "benzenesulfonyl groups that have undergone substitution with 1 to 4 nitro groups", and the like.

More specific examples of the tetrahydropyranyl groups or tetrahydrothiopyranyl groups include a tetrahydropyran-2-yl group, a 3-bromotetrahydropyran-2-yl group, a 4-methoxytetrahydropyran-4-yl group, a tetrahydrothiopyran-4-yl group, a 4-methoxytetrahydrothiopyran-4-yl group, and the like. Examples of the tetrahydrofuranyl groups or tetrahydrothiofuranyl groups include a tetrahydrofuran-2-yl group and a tetrahydrothiofuran-2-yl group. Examples of the lower-alkoxy-methyl groups include a methoxymethyl group, a 1,1-dimethyl-1-methoxymethyl group, an ethoxymethyl group, a propoxymethyl group, an isopropoxymethyl group, a butoxymethyl group, a t-butoxymethyl group, and the like. Examples of the low-alkoxylated lower-alkoxy-methyl groups include a 2-methoxyethoxymethyl group and the like. Examples of the halogeno lower-alkoxy-methyl groups include a 2,2,2-trichloroethoxymethyl group, a bis(2-chloroethoxy)methyl group, and the like. Examples of the low-alkoxylated ethyl groups include a 1-ethoxyethyl group, a 1-(isopropoxy)ethyl group, and the like. Examples of the halogenated ethyl groups include a 2,2,2-trichloroethyl group and the like. Examples of the methyl groups that have undergone substitution with 1 to 3 aryl groups include a benzyl group, an α-naphthylmethyl group, a β-naphthylmethyl group, a diphenylmethyl group, a triphenylmethyl group, an α-naphthyldiphenylmethyl group, a 9-anthrylmethyl group, and the like. Examples of the "methyl groups that have undergone substitution with 1 to 3 aryl groups in which an aryl ring has undergone substitution by a lower alkyl group, lower alkoxy group, halogen atom, or cyano group" include a 4-methylbenzyl group, a 2,4,6-trimethylbenzyl group, a 3,4,5-trimethylbenzyl group, a 4-methoxybenzyl group, a 4-methoxyphenyldiphenylmethyl group, a 4,4'-dimethoxytriphenylmethyl group, a 2-nitrobenzyl group, a 4-nitrobenzyl group, a 4-chlorobenzyl group, a 4-bromobenzyl group, a 4-cyanobenzyl group, and the like. Examples of the lower-alkoxy-carbonyl groups include a methoxycarbonyl group, an ethoxycarbonyl group, a t-butoxycarbonyl group, an isobutoxycarbonyl group, and the like. Examples of the "aryl groups that have undergone substitution with a halogen atom, lower alkoxy group, or nitro group" include a 4-chlorophenyl group, a 2-fluorophenyl group, a 4-methoxyphenyl group, a 4-nitrophenyl group, a 2,4-dinitrophenyl group, and the like. Examples of the "lower-alkoxy-carbonyl groups that have undergone substitution with a halogen atom or tri-loweralkyl-silyl group" include a 2,2,2-trichloroethoxycarbonyl group, 2-trimethylsilylethoxycarbonyl group, and the like. Examples of the alkenyloxycarbonyl groups include a vinyloxycarbonyl group, an aryloxycarbonyl group, and the like. Examples of the "aralkyloxycarbonyl groups in which an aryl ring may have undergone substitution with a lower alkoxy group or nitro group" include a benzyloxycarbonyl group, a 4-methoxybenzyloxycarbonyl group, a 3,4-dimethoxybenzyloxycarbonyl group, a 2-nitrobenzyloxycarbonyl group, a 4-nitrobenzyloxycarbonyl group, and the like. Examples of the "lower-alkoxy-carbonyl groups that have undergone substitution with a cyano group" include a cyanoethoxycarbonyl group and the like. Examples of the "benzenesulfonyl groups that have undergone substitution with 1 to 4 nitro groups" include a 2-nitrobenzenesulfonyl group, a 2,4-dinitrobenzenesulfonyl group, and the like.

The "hydroxy group protecting group for nucleic acid synthesis" is favorably an aliphatic acyl group, an aromatic acyl group, a methyl group that has undergone substitution with 1 to 3 aryl groups, a "methyl group that has undergone substitution with 1 to 3 aryl groups in which an aryl ring has undergone substitution with a lower alkyl, lower alkoxy, halogen, or cyano group", or a silyl group, and more favorably an acetyl group, a benzoyl group, a benzyl group, a p-methoxybenzoyl group, a dimethoxytrityl group, a monomethoxytrityl group, or a tert-butyldiphenylsilyl group. The protecting group used for the "hydroxy group protected by a protecting group for nucleic acid synthesis" is favorably an aliphatic acyl group, an aromatic acyl group, a "methyl group that has undergone substitution with 1 to 3 aryl groups", an "aryl group that has undergone substitution with a halogen atom, lower alkoxy group, or nitro group", a lower alkyl group, or a lower alkenyl group, and more favorably a benzoyl group, a benzyl group, a 2-chlorophenyl group, a 4-chlorophenyl group, or a 2-propenyl group. The "amino group protecting group for nucleic acid synthesis" is favorably an acyl group, and more favorably a benzoyl group. The "protecting group" used for the "phosphate group protected by a protecting group for nucleic acid synthesis" is favorably a lower alkyl group, a lower alkyl group that has undergone substitution with a cyano group, an aralkyl group, an "aralkyl group in which an aryl ring has undergone substitution with a nitro group or halogen atom", or an "aryl group that has undergone substitution with a lower alkyl group, halogen atom, or nitro group", and more favorably a 2-cyanoethyl group, a 2,2,2-trichloroethyl group, a benzyl group, a 2-chlorophenyl group, or a 4-chlorophenyl group. One or more protecting groups can be used for the "phosphate group protected by a protecting group for nucleic acid synthesis". The "protecting group" used for the "mercapto group protected by a protecting group for nucleic acid synthesis" is favorably an aliphatic acyl group or an aromatic acyl group, and more favorably a benzoyl group.

Examples of the "amino group protecting group" for the R¹⁰ group in this specification include an acetyl group, a tertiary butoxycarbonyl (Boc) group, a 9-fluorenylmethyloxycarbonyl (Fmoc) group, and the like.

In this specification, among groups represented by -P(R²⁴)R²⁵ (where R²⁴ and R²⁵ independently represent a hydroxy group, a hydroxy group protected by a protecting group for nucleic acid synthesis, a mercapto group, a mercapto group protected by a protecting group for nucleic acid synthesis, an amino group, a linear or branched alkoxy group having 1 to 6 carbon atoms, a linear or branched alkylthio group having 1 to 6 carbon atoms, a cyanoalkoxy group having 1 to 6 carbon atoms, or a linear or branched alkylamino group having 1 to 6 carbon atoms), a group in which R²⁴ can be represented as -OR^{24a} and R²⁵ can be represented as -N(R^{25a})₂ is referred to as a "phosphoramidite group". Favorable examples of the phosphoramidite group include a group represented by a formula -P(OC₂H₄CN)(N(iPr)₂) and a group represented by a formula -P(OCH₃)(N(iPr)₂). In these formulae, iPr represents an isopropyl group.

Hereinafter, the present invention will be described in detail.

### Oligonucleotide or Pharmacologically Acceptable Salt Thereof

An oligonucleotide or pharmacologically acceptable salt thereof of the present invention encompasses, for example, an antisense oligonucleotide (ASO) targeting the REST gene and a pharmacologically acceptable salt thereof.

Regarding the REST gene, the base sequence information of the human REST gene is available from NCBI Reference Sequence: NG_029447.1 Homo sapiens RE1 silencing transcription factor (REST), RefSeqGene on chromosome 4.

The antisense oligonucleotide (ASO) according to the present invention is an oligonucleotide capable of suppressing the expression of mRNA of the target gene.

The oligonucleotide of the present invention includes a nucleotide sequence complementary to a continuous sequence in at least a portion of the target region included in the base sequence of SEQ ID NO: 1. This target region refers to a region that can regulate the expression of the REST, and an example of the regulation is suppression of the REST expression itself. In the present invention, the target region included in the base sequence of SEQ ID NO: 1 is constituted by, for example, a continuous sequence having at least 15 bases, and has, for example, 15, 16, 17, 18, 19, 20, 21, 22, 23, 24, 25, 26, 27, 28, 29, 30, 31, 32, 33, 34 or 35 bases, but the number of bases in the region is not limited thereto.

The oligonucleotide (e.g., antisense oligonucleotide) of the present invention has at least 15 bases (e.g., 15, 16, 17, 18, 19, 20, 21, 22, 23, 24, 25, 26, 27, 28, 29, 30, 31, 32, 33, 34, or 35 bases), and preferably has 16 to 25 bases. It is possible to more effectively express the REST due to the number of bases in the oligonucleotide of the present invention being as described above.

Also, the oligonucleotide (e.g., antisense oligonucleotide) of the present invention preferably includes a sequence complementary to a target region between positions 900 and 4700 of SEQ ID NO: 1, more preferably between positions 2000 and 4650, and even more preferably between positions 2200 and 4100 and/or between positions 4600 and 4640, among the target regions included in the base sequence of SEQ ID NO: 1. In one embodiment, the oligonucleotide (e.g., antisense oligonucleotide) of the present invention includes a sequence complementary to the target region having a continuous sequence with at least 15 bases in the base sequences between positions 923 and 939, positions 1522 and 1540, positions 2248 and 2264, positions 4082 and 4101, and positions 4629 and 4651 of SEQ ID NO: 1. In one embodiment, the 5' terminus of the target region targeted by the oligonucleotide (e.g., antisense oligonucleotide) of the present invention corresponds to position 923, 1522, 1523, 1524, 2248, 4082, 4083, 4084, 4085, 4629, 4631, 4632, 4633, 4634, 4635, or 4645 of SEQ ID NO: 1. A method that is commonly used by a person skilled in the art can be used to design the sequence of an antisense oligonucleotide based on such a selected target region.

The following sequences (bases are aligned in a direction from 5' toward 3') are examples of the base sequence of the antisense oligonucleotide (target regions are shown together as "5'-terminal position - 3'-terminal position" using the numbers of the base positions in SEQ ID NO: 1):
cgttaggcagggccatt (SEQ ID NO: 2) (923-939);
atatcgattagtattgt (SEQ ID NO: 3) (1522-1538);
catatcgattagtattg (SEQ ID NO: 4) (1523-1539);
tcatatcgattagtatt (SEQ ID NO: 5) (1524-1540);
tttctctgctttgacgg (SEQ ID NO: 6) (2248-2264);
ggcgattgaggtgtttg (SEQ ID NO: 7) (4082-4098);
tggcgattgaggtgttt (SEQ ID NO: 8) (4083-4099);
atggcgattgaggtgtt (SEQ ID NO: 9) (4084-4100);
aatggcgattgaggtgt (SEQ ID NO: 10) (4085-4101);
tgtctattgctggggga (SEQ ID NO: 11) (4629-4645);
actgtctattgctgggg (SEQ ID NO: 12) (4631-4647);
gactgtctattgctggg (SEQ ID NO: 13) (4632-4648);
agactgtctattgctgg (SEQ ID NO: 14) (4633-4649);
tagactgtctattgctg (SEQ ID NO: 15) (4634-4650);
atagactgtctattgct (SEQ ID NO: 16) (4635-4651); and
acttggactgatagact (SEQ ID NO: 17) (4645-4661).

One to several bases (e.g., two or three bases) may be added to the 5' terminus and/or 3' terminus of the sequence above as long as the oligonucleotide can bind to the REST gene and has activity capable of suppressing the expression of the REST. These additional bases can be complementary to the bases of a sequence, shown in SEQ ID NO: 1, adjacent to the target region having a sequence to which the bases are to be added.

The oligonucleotide according to the present invention encompasses an oligonucleotide containing a chemically modified DNA. Such a modification can change the activity of the oligonucleotide and can improve, for example, the affinity for a target nucleic acid or the tolerance to a nucleic acid degradation enzyme (nuclease). Improving the affinity of the oligonucleotide for a target makes it possible to enable the use of a shorter oligonucleotide.

The oligonucleotide according to the present invention includes at least one sugar-modified nucleoside at any position. This sugar-modified nucleoside includes a cross-link as described below, for example, between positions 2 and 4 in the sugar ring.

In one embodiment, the oligonucleotide according to the present invention includes at least one nucleoside structure represented by Formula (I) below as a sugar-modified nucleoside:

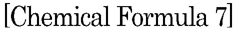

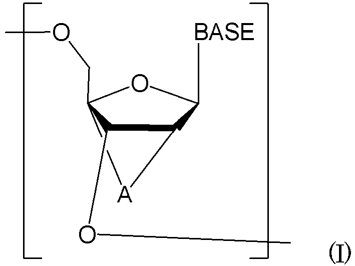

(In Formula (I),
BASE represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms, and
A is a divalent group represented by: 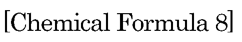 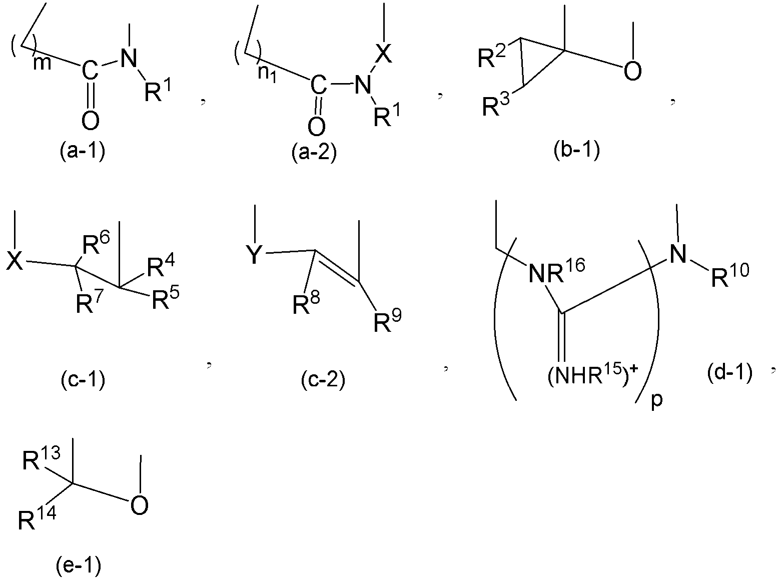 or
where
R¹ represents a hydrogen atom, an alkyl group having 1 to 7 carbon atoms that may be branched or cyclic, an alkenyl group having 2 to 7 carbon atoms that may be branched or cyclic, an aryl group having 3 to 12 carbon atoms that may have any one or more substituents selected from the α group and have a hetero atom, an aralkyl group with an aryl moiety having 3 to 12 carbon atoms that may have any one or more substituents selected from the α group and may have a hetero atom, or an amino group protecting group for nucleic acid synthesis;
R² and R³ are independently a hydrogen atom, an alkyl group having 1 to 7 carbon atoms that may have undergone substitution with an aryl group having 3 to 12 carbon atoms that may have a hetero atom, and may be branched or cyclic, or an aralkyl group with an aryl moiety having 3 to 12 carbon atoms that may have a hetero atom, or
R² and R³ together represent -(CH₂)_{q}- (where q is an integer from 2 to 5);
R⁴ and R⁵ are independently a group selected from the group consisting of a hydrogen atom, a hydroxy group, alkyl groups having 1 to 7 carbon atoms that may be branched or cyclic, alkoxy groups having 1 to 7 carbon atoms that may be branched or cyclic, an amino group, and an amino group protected by a protecting group for nucleic acid synthesis, or R⁴ and R⁵ together form =C(R¹¹)R¹² (where R¹¹ and R¹² independently represent a hydrogen atom, a hydroxy group, a hydroxy group protected by a protecting group for nucleic acid synthesis, a mercapto group, a mercapto group protected by a protecting group for nucleic acid synthesis, an amino group, a linear or branched alkoxy group having 1 to 6 carbon atoms, a linear or branched alkylthio group having 1 to 6 carbon atoms, a cyanoalkoxy group having 1 to 6 carbon atoms, or a linear or branched alkylamino group having 1 to 6 carbon atoms);
R⁶ and R⁷ are independently a hydrogen atom, an alkyl group having 1 to 7 carbon atoms that may be branched or cyclic, an alkoxy group having 1 to 7 carbon atoms that may be branched or cyclic, or a linear or branched alkylthio group having 1 to 6 carbon atoms;
R⁸ represents a hydrogen atom, an alkyl group having 1 to 7 carbon atoms that may be branched or cyclic, an alkoxy group having 1 to 7 carbon atoms that may be branched or cyclic, or a linear or branched alkylthio group having 1 to 6 carbon atoms;
R⁹ is a hydrogen atom, a hydroxy group, an alkyl group having 1 to 7 carbon atoms that may be branched or cyclic, an alkoxy group having 1 to 7 carbon atoms that may be branched or cyclic, an amino group, or an amino group protected by a protecting group for nucleic acid synthesis;
R¹⁰ is a hydrogen atom, an alkyl group having 1 to 7 carbon atoms that may be branched or cyclic, an amino group protecting group, or 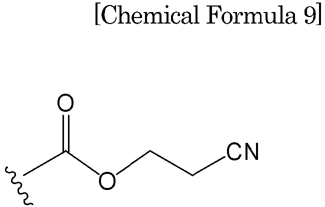
[0076] or -(C=(NHR¹⁷)⁺)-NR¹⁸R¹⁹ (where R¹⁷, R¹⁸, and R¹⁹ are independently a hydrogen atom, an alkyl group having 1 to 7 carbon atoms that may be branched or cyclic, an amino group protecting group, or 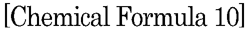 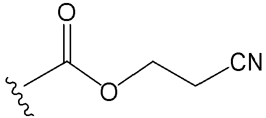
or -(C=(NHR¹⁷)⁺)-NR¹⁸R¹⁹ (where R¹⁷, R¹⁸, and R¹⁹ are independently a hydrogen atom, an alkyl group having 1 to 7 carbon atoms that may be branched or cyclic, an amino group protecting group, or 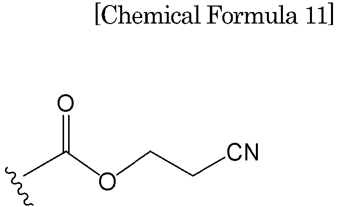 );
R¹³ and R¹⁴ are independently a group selected from the group consisting of a hydrogen atom, a hydroxy group, alkyl groups having 1 to 7 carbon atoms that may be branched or cyclic, alkoxy groups having 1 to 7 carbon atoms that may be branched or cyclic, an amino group, and an amino group protected by a protecting group for nucleic acid synthesis;
m is an integer from 0 to 2;
n is an integer of 0 or 1;
when R¹⁰ is a hydrogen atom, an alkyl group having 1 to 7 carbon atoms that may be branched or cyclic, an amino group protecting group, or 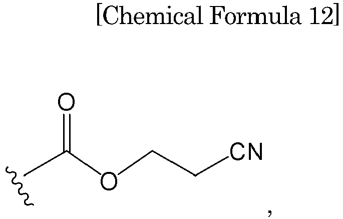
p is 1, and R¹⁵ and R¹⁶ are independently a hydrogen atom, an alkyl group having 1 to 7 carbon atoms that may be branched or cyclic, an amino group protecting group, or 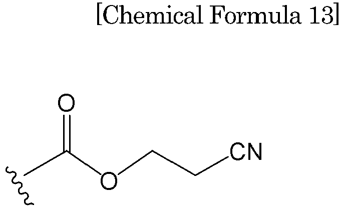 or
when R¹⁰ is -(C=(NHR¹⁷)⁺)-NR¹⁸R¹⁹, p is 0;
X is an oxygen atom, a sulfur atom, or an amino group; and
Y is an oxygen atom or a sulfur atom).

In one embodiment, the nucleoside structure represented by Formula (I) above is a structure represented by the following formula: 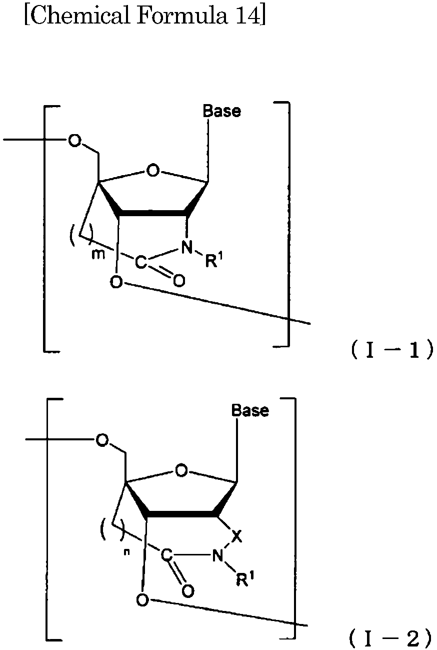 or

Base, R¹, X, m, and n in Formulae (I-1) and (I-2) are the same as those in Formula (I) described above. An amide (-CONR¹-) is introduced into the cross-link between positions 2' and 4', and such a nucleoside structure is also referred to as an "amide bridged nucleic acid", an "amide BNA (Bridged Nucleic Acid)", or "AmNA".

In Formulae (I-1) and (I-2), R¹ is a hydrogen atom, an alkyl group having 1 to 7 carbon atoms that may be branched or cyclic, an alkenyl group having 2 to 7 carbon atoms that may be branched or cyclic, an aryl group having 3 to 12 carbon atoms that may have any one or more substituents selected from the α group and may have a hetero atom, or an aralkyl group with an aryl moiety having 3 to 12 carbon atoms that may have any one or more substituents selected from the α group and may have a hetero atom. R¹ is more favorably a hydrogen atom, a methyl group, an ethyl group, an n-propyl group, an isopropyl group, a phenyl group, or a benzyl group, and even more favorably a hydrogen atom or a methyl group.

In Formula (I-1), m is an integer from 0 to 2, and in Formula (I-2), n is an integer of 0 or 1. That is to say, a ring that includes positions 2', 3', and 4', and a cross-linked portion is a five- to seven-membered ring.

In Formula (I-2), X is an oxygen atom, a sulfur atom, an amino group, or a methylene group. X is favorably an oxygen atom or an amino group. When X is an amino group or a methylene group, X may have undergone substitution with a lower alkyl group.

In one embodiment, the nucleoside structure represented by Formula (I) above is a structure represented by Formula (I-1) above, and in this Formula (I-1), m is 0, and R¹ is a hydrogen atom, a methyl group, an ethyl group, an n-propyl group, an isopropyl group, a phenyl group, or a benzyl group.

In the compounds represented by Formulae (I-1) and (I-2), an amide bond is formed between the amino group at position 2' and the carbonyl group extending from position 4' in a sugar moiety. An amide bond, which has little structural fluctuation and excellent hydrophilicity, is provided, and therefore, the structure of the sugar moiety in the nucleoside is fixed by the cross-link.

Examples of the nucleoside structure represented by Formula (I) above include those represented by Formulae (I-3) to (I-7) below in addition to those represented by Formulae (I-1) and (I-2) above:

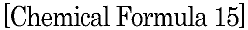

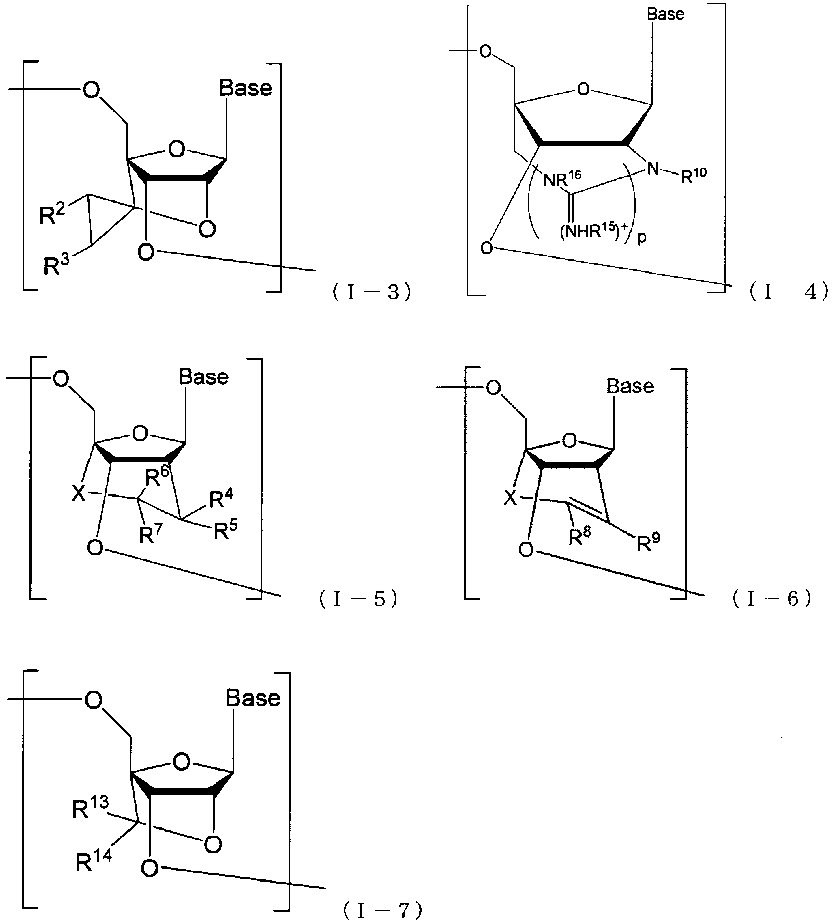

In the formulae above, Base, R², R³, R⁴, R⁵, R⁶, R⁷, R⁸, R⁹, R¹⁰, R¹³, R¹⁴, R¹⁵, R¹⁶, and p are the same as those in Formula (I) described above. Formula (I-7) corresponds to a nucleoside structure called a 2',4'-BNA or an LNA (Locked Nucleic Acid) (also referred to as a "2',4'-BNA/LNA" or "LNA" in this specification) (in one example, both R¹³ and R¹⁴ are hydrogen atoms). Formula (I-3) shows a structure obtained by introducing a spirocyclopropane group into position 6' of the cross-link structure of a 2',4'-BNA/LNA, and this structure is also called a spirocyclopropane bridged nucleic acid (scpBNA). Formula (I-4) shows a structure obtained by introducing a guanidine into the cross-link structure of a 2',4'-BNA/LNA, and this structure is also called a guanidine bridged nucleic acid (GuNA). Note that Formula (I-4) encompasses Formulae (I-4-1) (p = 0) and (I-4-2) (p = 1) below:

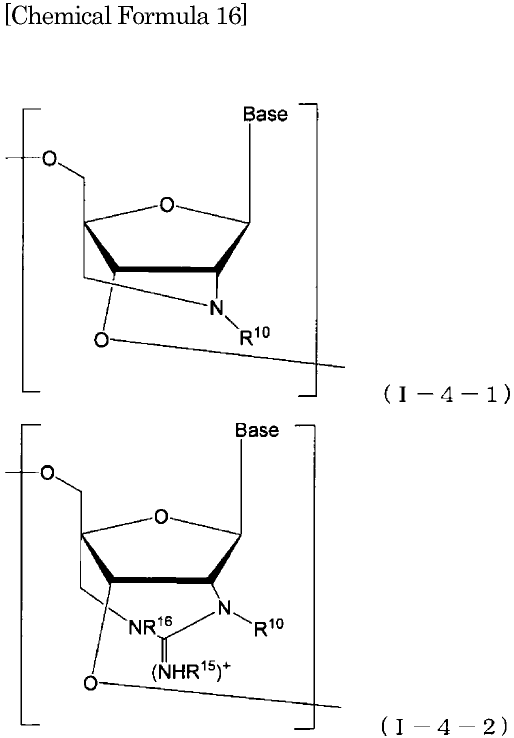

"Base" above is a purine base (i.e., purin-9-yl group) or a pyrimidine base (i.e., 2-oxo-1,2-dihydropyrimidin-1-yl group). These bases may have any one or more substituents selected from the α group consisting of a hydroxy group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms, and halogen atoms.

Specific examples of "Base" above include an adeninyl group, a guaninyl group, a cytosinyl group, a uracilyl group, a thyminyl group, a 6-aminopurin-9-yl group, a 2,6-diaminopurin-9-yl group, a 2-amino-6-chloropurin-9-yl group, a 2-amino-6-fluoropurin-9-yl group, a 2-amino-6-bromopurin-9-yl group, a 2-amino-6-hydroxypurin-9-yl group, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl group, a 4-amino-2-oxo-5-fluoro-1,2-dihydropyrimidin-1-yl group, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl group, a 2-oxo-4-hydroxy-5-methyl-1,2-dihydropyrimidin-1-yl group, and a 4-amino-5-methyl-2-oxo-1,2-dihydropyrimidin-1-yl group.

In particular, as "Base", groups represented by structural formulae below:

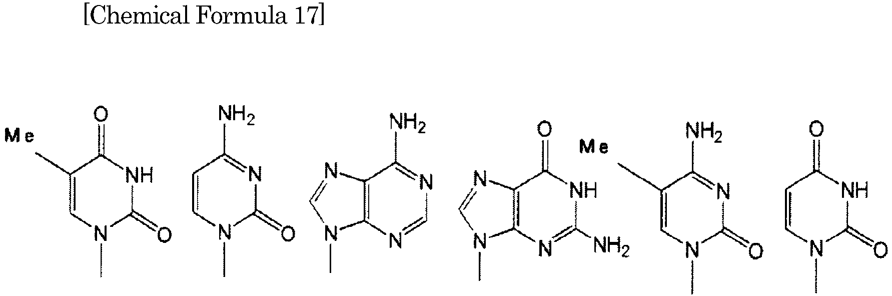

(i.e., a thyminyl group, a cytosinyl group, an adeninyl group, a guaninyl group, a 5-methylcytosinyl group, and an uracilyl group), and a 2-oxo-4-hydroxy-5-methyl-1,2-dihydropyrimidin-1-yl group, a 2-oxo-4-amino-1,2-dihydropyrimidin-1-yl group, a 6-aminopurin-9-yl group, a 2-amino-6-hydroxypurin-9-yl group, a 4-amino-5-methyl-2-oxo-1,2-dihydropyrimidin-1-yl group, and a 2-oxo-4-hydroxy-1,2-dihydropyrimidin-1-yl group are favorable, and a 2-oxo-4-hydroxy-5-methyl-1,2-dihydropyrimidin-1-yl group and a thyminyl group are particularly favorable. It is preferable that a hydroxy group and an amino group are protected by a protecting group during oligonucleotide synthesis.

In one embodiment, when the oligonucleotide according to the present invention includes, as a sugar-modified nucleoside, the nucleoside structure represented by Formula (I-4) or Formulae (I-4-1) or (I-4-2) above, the nucleoside structure represented by Formula (I-4) or Formulae (I-4-1) or (I-4-2) is kept electrically neutral by an anion (e.g., represented as Z⁻) that is not shown in Formula (I-4) or Formulae (I-4-1) or (I-4-2). Examples of such an anion include halide ions (e.g., chloride ion), a phosphate ion, and the like.

The oligonucleotide that includes at least one sugar-modified nucleoside structure as described above can be synthesized using, for example, the methods as described in WO 2011/052436, JP 2014-043462A, WO 2014/046212, and WO 2015/125783 by use of, for example, a sugar-modified nucleoside compound.

Examples of the sugar-modified nucleoside compound include compounds represented by Formula (II) below or salts thereof: 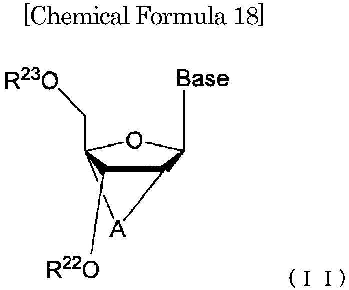 (where
Base represents a purin-9-yl group that may have any one or more substituents selected from an α group, or a 2-oxo-1,2-dihydropyrimidin-1-yl group that may have any one or more substituents selected from the α group, the α group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms, and
A is a divalent group represented by: 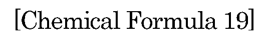 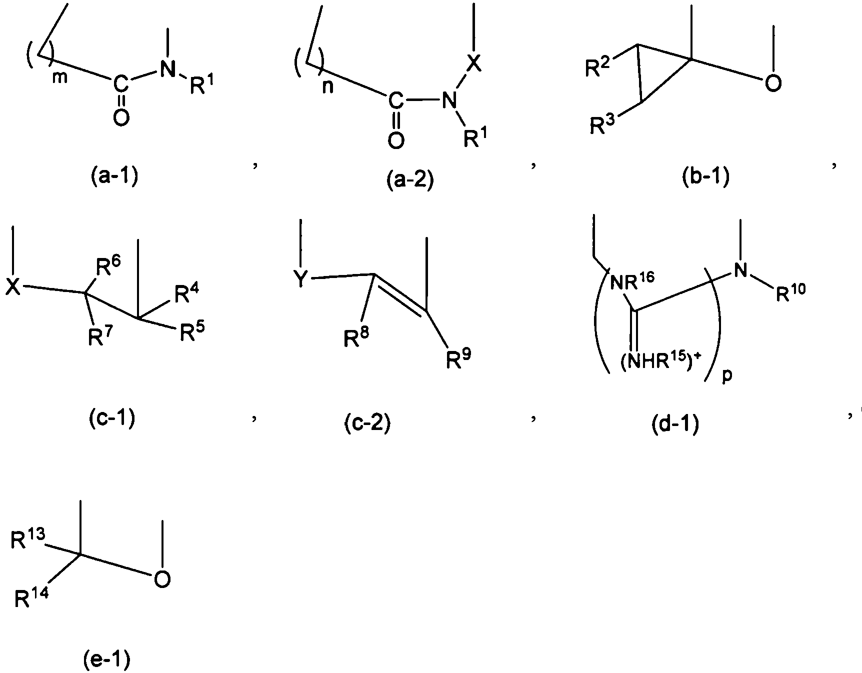 or
where
R¹ represents a hydrogen atom, an alkyl group having 1 to 7 carbon atoms that may be branched or cyclic, an alkenyl group having 2 to 7 carbon atoms that may be branched or cyclic, an aryl group having 3 to 12 carbon atoms that may have any one or more substituents selected from the α group and have a hetero atom, an aralkyl group with an aryl moiety having 3 to 12 carbon atoms that may have any one or more substituents selected from the α group and may have a hetero atom, or an amino group protecting group for nucleic acid synthesis;
R² and R³ are independently a hydrogen atom, an alkyl group having 1 to 7 carbon atoms that may have undergone substitution with an aryl group having 3 to 12 carbon atoms that may have a hetero atom, and may be branched or cyclic, or an aralkyl group with an aryl moiety having 3 to 12 carbon atoms that may have a hetero atom, or
R² and R³ together represent -(CH₂)_{q}- (where q is an integer from 2 to 5);
R⁴ and R⁵ are independently a group selected from the group consisting of a hydrogen atom, a hydroxy group, alkyl groups having 1 to 7 carbon atoms that may be branched or cyclic, alkoxy groups having 1 to 7 carbon atoms that may be branched or cyclic, an amino group, and an amino group protected by a protecting group for nucleic acid synthesis, or R⁴ and R⁵ together form =C(R¹¹)R¹² (where R¹¹ and R¹² independently represent a hydrogen atom, a hydroxy group, a hydroxy group protected by a protecting group for nucleic acid synthesis, a mercapto group, a mercapto group protected by a protecting group for nucleic acid synthesis, an amino group, a linear or branched alkoxy group having 1 to 6 carbon atoms, a linear or branched alkylthio group having 1 to 6 carbon atoms, a cyanoalkoxy group having 1 to 6 carbon atoms, or a linear or branched alkylamino group having 1 to 6 carbon atoms);
R⁶ and R⁷ are independently a hydrogen atom, an alkyl group having 1 to 7 carbon atoms that may be branched or cyclic, an alkoxy group having 1 to 7 carbon atoms that may be branched or cyclic, or a linear or branched alkylthio group having 1 to 6 carbon atoms;
R⁸ represents a hydrogen atom, an alkyl group having 1 to 7 carbon atoms that may be branched or cyclic, an alkoxy group having 1 to 7 carbon atoms that may be branched or cyclic, or a linear or branched alkylthio group having 1 to 6 carbon atoms;
R⁹ is a hydrogen atom, a hydroxy group, an alkyl group having 1 to 7 carbon atoms that may be branched or cyclic, an alkoxy group having 1 to 7 carbon atoms that may be branched or cyclic, an amino group, or an amino group protected by a protecting group for nucleic acid synthesis;
R¹⁰ is a hydrogen atom, an alkyl group having 1 to 7 carbon atoms that may be branched or cyclic, an amino group protecting group, or 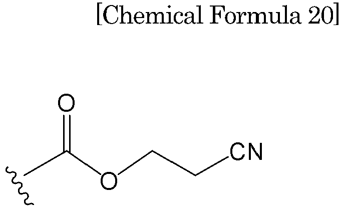
or -(C=(NHR¹⁷)⁺)-NR¹⁸R¹⁹ (where R¹⁷, R¹⁸, and R¹⁹ are independently a hydrogen atom, an alkyl group having 1 to 7 carbon atoms that may be branched or cyclic, an amino group protecting group, or 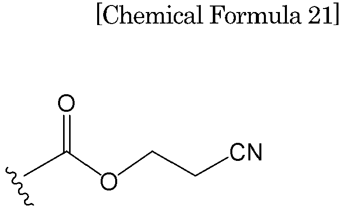 );
R¹³ and R¹⁴ are independently a group selected from the group consisting of a hydrogen atom, a hydroxy group, alkyl groups having 1 to 7 carbon atoms that may be branched or cyclic, alkoxy groups having 1 to 7 carbon atoms that may be branched or cyclic, an amino group, and an amino group protected by a protecting group for nucleic acid synthesis;
m is an integer from 0 to 2;
n is an integer of 0 or 1;
when R¹⁰ is a hydrogen atom, an alkyl group having 1 to 7 carbon atoms that may be branched or cyclic, an amino group protecting group, or 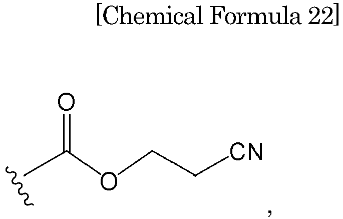
p is 1, and R¹⁵ and R¹⁶ are independently a hydrogen atom, an alkyl group having 1 to 7 carbon atoms that may be branched or cyclic, an amino group protecting group, or 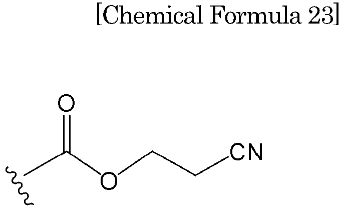 or
when R¹⁰ is -(C=(NHR¹⁷)⁺)-NR¹⁸R¹⁹ (where R¹⁷, R¹⁸, and R¹⁹ are independently a hydrogen atom, an alkyl group having 1 to 7 carbon atoms that may be branched or cyclic, an amino group protecting group, or 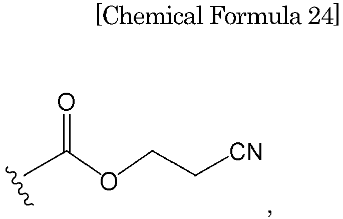
p is 0;
X is an oxygen atom, a sulfur atom, or an amino group;
Y is an oxygen atom or a sulfur atom; and
R²² and R²³ each independently represent a hydrogen atom, a hydroxy group protecting group for nucleic acid synthesis, an alkyl group having 1 to 7 carbon atoms that may be branched or cyclic, an alkenyl group having 2 to 7 carbon atoms that may be branched or cyclic, an aryl group having 3 to 12 carbon atoms that may have any one or more substituents selected from the α group and may have a hetero atom, an aralkyl group with an aryl moiety having 3 to 12 carbon atoms that may have any one or more substituents selected from the α group and may have a hetero atom, an acyl group that may have any one or more substituents selected from the α group, a silyl group that may have any one or more substituents selected from the α group, a phosphate group that may have any one or more substituents selected from the α group, a phosphate group protected by a protecting group for nucleic acid synthesis, or -P(R²⁴)R²⁵ (where R²⁴ and R²⁵ independently represent a hydroxy group, a hydroxy group protected by a protecting group for nucleic acid synthesis, a mercapto group, a mercapto group protected by a protecting group for nucleic acid synthesis, an amino group, an alkoxy group having 1 to 5 carbon atoms, an alkylthio group having 1 to 5 carbon atoms, a cyanoalkoxy group having 1 to 6 carbon atoms, or a dialkylamino group that has undergone substitution with an alkyl group having 1 to 6 carbon atoms).

A sugar-modified nucleotide can be easily prepared using the sugar-modified nucleosides as described above. For example, triphosphorylation can be easily conducted in accordance with the method described in M. Kuwahara et al., Nucleic Acids Res., 2008, vol. 36, No. 13, pp. 4257-65.

The nucleotide modifications known in the art other than the above-described modification to sugar can also be used. A modification to phosphate and a modification to a nucleic acid base are known as the nucleotide modifications. Such modifications to a nucleic acid can be conducted based on methods known in the art.

Examples of the modification to phosphate include a phosphodiester bond included in a natural nucleic acid, S-oligo (phosphorothioate), D-oligo (phosphodiester), M-oligo (methylphosphonate), boranophosphate, and the like. The S-oligo (phosphorothioate) has a PS skeleton obtained by substituting oxygen atoms in the phosphate group moieties in the phosphodiester bonds between the nucleosides with sulfur atoms. This modification is incorporated into oligonucleotides according to a known method. An antisense oligonucleotide that includes this modification at one or more positions in the oligonucleotide is referred to as an S-oligo type (phosphorothioate type).

Examples of the modification to a nucleic acid base include 5-methylcytosine, 5-hydroxymethylcytosine, 5-propynylcytosine, and the like.

There is no particular limitation on the positions and number of the sugar modified nucleosides in the oligonucleotide of the present invention, and the oligonucleotide can be designed as appropriate depending on the purpose. Two or more sugar-modified nucleosides may be the same or different.

It is preferable that the oligonucleotide of the present invention is a gapmer (particularly when the oligonucleotide is a single-stranded oligonucleotide). The "gapmer" means an oligonucleotide that includes a "gap", which is a central region, and two wings, which are regions located on both sides of the gap, namely a "5' wing" located on the 5' side and a "3' wing" located on the 3' side. The gap region can have 9 to 15 bases, and the wing regions can have 3 to 5 bases. The gap is constituted by natural nucleosides, and the wings can include at least one modified nucleotide. The oligonucleotide of the present invention includes at least one sugar-modified nucleoside, preferably 1 to 5 sugar-modified nucleosides, in the "5' wing" and/or the "3' wing". In one embodiment, the gapmer can include a gap region having 9 to 13 bases, a 5' wing having 3 to 5 bases, and a 3' wing having 3 to 5 bases, the gap region is located between the 5' wing and the 3' wing, and the 5' wing and the 3' wing each can have at least one nucleoside structure represented by Formula (I) above. In addition, the gapmer may include a modification to phosphate, a modification to a base, and the like. The types, number, and positions of modifications in one wing may be the same as or different from those in the other wing. The "wings" of the gapmer may be a wing in which all the bases included are modified nucleotides, or a wing in which some of the bases included are natural nucleosides, and also encompass a wing in which one base at the 3' terminus of the 3' wing is a natural nucleoside (e.g., DNA).

In one embodiment, the oligonucleotide according to the present invention is an oligonucleotide that includes a base sequence represented by any of the base sequences of SEQ ID NOs: 2 to 17 above and in which at least one of the bases is the sugar-modified nucleoside above. The following are examples of such an oligonucleotide:
REST-923-17(L) (SEQ ID NO: 18),
REST-1522-17(L) (SEQ ID NO: 19),
REST-1523-17(L) (SEQ ID NO: 20),
REST-1524-17(L) (SEQ ID NO: 21),
REST-2248-17(L) (SEQ ID NO: 22),
REST-4082-17(L) (SEQ ID NO: 23),
REST-4083-17(L) (SEQ ID NO: 24),
REST-4084-17(L) (SEQ ID NO: 25),
REST-4085-17(L) (SEQ ID NO: 26),
REST-4629-17(L) (SEQ ID NO: 27),
REST-4631-17(L) (SEQ ID NO: 28),
REST-4632-17(L) (SEQ ID NO: 29),
REST-4633-17(L) (SEQ ID NO: 30),
REST-4634-17(L) (SEQ ID NO: 31),
REST-4635-17(L) (SEQ ID NO: 32),
REST-4645-17(L) (SEQ ID NO: 33),
REST-2248-17(Y) (SEQ ID NO: 34),
REST-4082-17(Y) (SEQ ID NO: 35), and
REST-4633-17(Y) (SEQ ID NO: 36).

Here, regarding the oligonucleotides listed above, "(L)" indicates that the oligonucleotide includes a nucleic acid called an "LNA" represented by Formula (a) below, and "(Y)" indicates that the oligonucleotide includes a nucleic acid called an "AmNA" represented by Formula (b) below.

The oligonucleotide and pharmacologically acceptable salt thereof according to the present invention can be synthesized using an ordinary method by use of the sugar-modified nucleosides as described above and natural nucleosides, and can be easily synthesized, for example, using a commercially available automated nucleic acid synthesizer (e.g., those manufactured by Applied Biosystems, GeneDesign Inc., and the like). Solid phase synthesis using phosphoroamidite, solid phase synthesis using hydrogen phosphonate, and the like are used as the synthesis method. For example, the methods disclosed in Tetrahedron Letters, 1981, vol. 22. pp. 1859-1862, WO 2011/052436, WO 2014/046212, WO 2015/125783, and the like can be used.

REST Expression Suppressing Agent and Pharmaceutical Composition

A REST expression suppressing agent of the present invention contains the oligonucleotide or pharmacologically acceptable salt thereof described above and can be used to suppress the expression of the REST gene in vivo or in vitro.

In one embodiment, the REST expression suppressing agent of the present invention is a pharmaceutical composition. That is to say, a pharmaceutical composition of the present invention contains the oligonucleotide or pharmacologically acceptable salt thereof described above. The pharmaceutical composition can be used to treat various nervous system diseases such as various nervous system diseases (e.g., malignant tumors, dementia, and central nervous system diseases) and diabetes, which are considered to be caused by the expression of the REST.

The pharmaceutical composition of the present invention can be administered using various methods depending on a topical or systemic treatment or regions to be treated. For example, topical administration (including ocular instillation, intravaginal administration, intrarectal administration, intranasal administration, and percutaneous administration), oral administration, or parenteral administration may be employed as the administration method. Examples of parenteral administration include intravenous injection, intravenous instillation, subcutaneous transfusion, intraabdominal transfusion, intramuscular transfusion, pulmonary administration via the airway through aspiration or inhalation, and the like.

The pharmaceutical composition of the present invention can be topically administered using formulations such as a percutaneous patch, ointment, lotion, cream, gel, drops, suppository, spray, liquid medicine, and powder medicine. Examples of compositions for oral administration include powder medicine, granular medicine, a suspension or solution obtained through dissolution in water or a non-aqueous medium, a capsule, powdered medicine, a tablet, and the like. Examples of compositions for parenteral administration include sterile aqueous solutions containing a buffer, a diluent, and other appropriate additives.

The REST expression suppressing agent and pharmaceutical composition of the present invention may contain, in addition to the oligonucleotide or pharmacologically acceptable salt thereof, various pharmaceutical additives suitable for the effective dose and/or the dosage form thereof, such as a vehicle, a binding agent, a moistening agent, a disintegrating agent, a lubricant, and a diluent, as needed. When the pharmaceutical composition is an injection, it may be sterilized together with an appropriate carrier.

The REST expression suppressing agent and pharmaceutical composition of the present invention can also be used for "individuals" of various types. The "individual" is preferably a mammal, more preferably a human, monkey, dog, cat, rat, or mouse, and even more preferably a human. The effective administration dose depends on the type, sex, age, weight, symptom and the like of the individual to which the REST expression suppressing agent or pharmaceutical composition of the present invention is to be administered, and an appropriate dose can be determined by a person skilled in the art in accordance with the method, route, frequency and the like of the administration.

### Examples

Hereinafter, the present invention will be described by way of examples, but the present invention is not limited thereto.

### Example 1: Oligonucleotide Synthesis

Oligonucleotides related to the present invention were synthesized using the methods described in Tetrahedron Letters 22, 1859-1862 (1981), WO 2011/052436, and the like.

Specifically, the synthesis of oligonucleotides containing a 2',4'-BNA/LNA represented by Formula (a) below was entrusted to GeneDesign Inc.

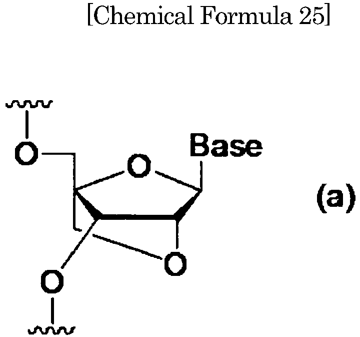

### (In the formula, Base is a 5-methylcytosinyl group, thyminyl group, adeninyl group, or guaninyl group.)

Oligonucleotides containing an amide BNA (AmNA) represented by Formula (b) below were synthesized with reference to the method described in WO 2011/052436.

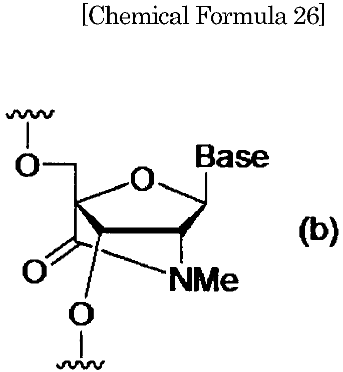

### (In the formula, Base is a 5-methylcytosinyl group, thyminyl group, adeninyl group, or guaninyl group, and Me is methyl.)

17-mer oligonucleotides containing a 2',4'-BNA/LNA represented by Formula (a) or an amide BNA (AmNA) represented by Formula (b) were synthesized at 0.2 µmol scale using an automated nucleic acid synthesizer (Type nS-8, manufactured by GeneDesign Inc.). The strand length was elongated in accordance with a standard phosphoroamidite protocol (solid phase support: CPG resin; DDT (3H-1,2-Benzodithiole-3-one,1,1-dioxide) or the like was used in sulfurization for forming a phosphorothioated (PS) skeleton), and thus an oligonucleotide in which a hydroxy group at the 5' end was protected by a DMTr (dimethoxytrityl) group and the 3' end was held on the solid phase was obtained. Next, the DMTr group was removed through acid treatment, and base treatment was conducted to remove target products from the solid phase support. After neutralization using dilute acid, the solvent was distilled off, and then the resultant crude product was purified using gel filtration column chromatography and reversed phase HPLC. The target products were thus obtained.

The cross-linked structure of the LNA used in this example and the purities and structures of the obtained oligonucleotides were confirmed using HPLC and MALDI-TOF-MS (manufactured by BRUKER DALTONICS).

### Example 2: Designing of Antisense Oligonucleotide for Human REST pre-mRNA

Antisense oligonucleotides were designed to target pre-mRNA (mRNA precursor) of the human REST gene (NCBI Reference Sequence: NG_029447.1 Homo sapiens RE1 silencing transcription factor (REST), RefSeqGene on chromosome 4. (SEQ ID NO: 1)).

In order to select target regions, the reverse sequences (CG, GGA, and GCA) of CG, TCC, and TGC were excluded because CG, TCC, and TGC are toxic in an antisense strand. Then, regions such as a loop structure that are easy for an antisense oligonucleotide to reach were selected based on the secondary structure predicted using mfold (mfold: http://unafold.rna.albany.edu/?q=mfold). Next, regions that are highly homologous between the human REST pre-mRNA and the mouse REST pre-mRNA were mainly selected using Blast (BLAST: https://blast.ncbi.nlm.nih.gov/Blast.cgi?PAGE_TYPE=BlastSearch) such that the evaluation results obtained from mice could be applied to humans. In this manner, candidate sequences were selected.

Oligonucleotides having base sequences complementary to the candidate sequences selected as described above were designed as gapmer-type antisense oligonucleotides (also referred to as "gapmer-type ASOs" hereinafter). Specifically, the length of each of the antisense oligonucleotides was set to 17 mer, artificial nucleic acid regions containing sugar-modified nucleosides corresponding to Formula (a) or (b) above were provided at the 5' terminus and the 3' terminus, and a natural nucleic acid region containing natural nucleosides (DNAs) was provided in the central portion. More specifically, a 3-11-3 gapmer was designed in which three bases on the 5' terminal side (5' wing region) were sugar-modified nucleosides, subsequent eleven bases (gap region) were natural nucleosides (DNAs), and subsequent three bases on the 3' terminal side (3' wing region) were sugar-modified nucleosides.

Table 1 shows the sequences (in the direction from 5' toward 3') of gapmer-type antisense oligonucleotides in which 2',4'-BNA/LNAs (indicated as "(L)" in Table 1) are used as sugar-modified nucleosides and the base positions in SEQ ID NO: 1 corresponding to the 5' termini and the 3' termini of the sequences of the target regions for the gapmer-type antisense oligonucleotides.

**Table 1**

| Name of Oligonucleotide | Antisense (5'→3') | Target Sequence | | SEQ ID NO. |
|---|---|---|---|---|
| | | 5' Termini | 3' Termini | |
| REST-923-17(L) | C(L)^G(L)^T(L)^t^a^g^g^c^a^g^g^g^c^c^A(L)^T(L)^T(L) | 923 | 939 | 18 |
| REST-1522-17(L) | A(L)^T(L)^A(L)^t^c^g^a^t^t^a^g^t^a^t^T(L)^G(L)^T(L) | 1522 | 1539 | 19 |
| REST-1523-17(L) | C(L)^A(L)^T(L)^a^t^c^g^a^t^t^a^g^t^a^T(L)^T(L)^G(L) | 1523 | 1539 | 20 |
| REST-1524-17(L) | T(L)^C(L)^A(L)^t^a^t^c^g^a^t^t^a^g^t^A(L)^T(L)^T(L) | 1524 | 1540 | 21 |
| REST⁻2248-17(L) | T(L)^T(L)^T(L)^c^t^c^t^g^c^t^t^t^g^a^C(L)^G(L)^G(L) | 2248 | 2264 | 22 |
| REST-4082-17(L) | G(L)^G(L)^C(L)^g^a^t^t^g^a^g^g^t^g^t^T(L)^T(L)^G(L) | 4082 | 4098 | 23 |
| REST-4083-17(L) | T(L)^G(L)^G(L)^c^g^a^t^t^g^a^g^g^t^g^T(L)^T(L)^T(L) | 4083 | 4099 | 24 |
| REST-4084-17(L) | A(L)^T(L)^G(L)^g^c^g^a^t^t^g^a^g^g^t^G(L)^T(L)^T(L) | 4084 | 4101 | 25 |
| REST-4085-17(L) | A(L)^A(L)^T(L)^g^g^c^g^a^t^t^g^a^g^g^T(L)^G(L)^T(L) | 4085 | 4101 | 26 |
| REST-4629-17(L) | T(L)^G(L)^T(L)^c^t^a^t^t^g^c^t^g^g^g^G(L)^G(L)^A(L) | 4629 | 4645 | 27 |
| REST-4631-17(L) | A(L)^C(L)^T(L)^g^t^c^t^a^t^t^g^c^t^g^G(L)^G(L)^G(L) | 4631 | 4647 | 28 |
| REST-4632-17(L) | G(L)^A(L)^C(L)^t^g^t^c^t^a^t^t^g^c^t^G(L)^G(L)^G(L) | 4632 | 4648 | 29 |
| REST-4633-17(L) | A(L)^G(L)^A(L)^c^t^g^t^c^t^a^t^t^g^c^T(L)^G(L)^G(L) | 4633 | 4649 | 30 |
| REST-4634-17(L) | T(L)^A(L)^G(L)^a^c^t^g^t^c^t^a^t^t^g^C(L)^T(L)^G(L) | 4634 | 4650 | 31 |
| REST-4635-17(L) | A(L)^T(L)^A(L)^g^a^c^t^g^t^c^t^a^t^t^G(L)^C(L)^T(L) | 4635 | 4651 | 32 |
| REST-4645-17(L) | A(L)^C(L)^T(L)^t^g^g^a^c^t^g^a^t^a^g^A(L)^C(L)^T(L) | 4645 | 4661 | 33 |

| | | | | |
|---|---|---|---|---|
| The 'A(L)', 'G(L)', 'C(L)' and 'T(L)' represent 2',4'-BNA/LNA bases. The 'a', 'g', 'c' and 't' represent DNA bases. The '^' denotes phosphorothioated (PS skeleton). | | | | |

Table 2 shows the sequences (in the direction from 5' toward 3') of gapmer-type antisense oligonucleotides in which AmNAs (indicated as "(Y)" in Table 2) are used as sugar-modified nucleosides and the base positions in SEQ ID NO: 1 corresponding to the 5' termini and the 3' termini of the sequences of the target regions for the gapmer-type antisense oligonucleotides.

**Table 2**

| Name of Oligonucleotide | Antisense (5'→3') | Target Sequence | | SEQ ID NO. |
|---|---|---|---|---|
| | | 5' Termini | 3' Termini | |
| REST-2248-17(Y) | T(Y)^T(Y)^T(Y)^c^t^c^t^g^c^t^t^t^g^a^C(Y)^G(Y)^G(Y) | 2248 | 2264 | 34 |
| REST-4082-17(Y) | G(Y)^G(Y)^C(Y)^g^a^t^t^g^a^g^g^t^g^t^T(Y)^T(Y)^G(Y) | 4082 | 4098 | 35 |
| REST-4633-17(Y) | A(Y)^G(Y)^A(Y)^c^t^g^t^c^t^a^t^t^g^c^T(Y)^G(Y)^G(Y) | 4633 | 4649 | 36 |

| | | | | |
|---|---|---|---|---|
| The 'A(Y)', 'G(Y)', 'C(Y)' and T(Y)' represent AmNA bases. The 'a', 'g', 'c' and '' represent DNA bases. The '^' denotes phosphorothioated (PS skeleton). | | | | |

Note that the term "phosphorothioated" means that an oxygen atom in a phosphate group in a phosphodiester bond is substituted with a sulfur atom (a group corresponding to a phosphate group is referred to as a "phosphorothioate group"). In this specification, an oligonucleotide in which all the phosphate groups in the oligonucleotide are substituted with phosphorothioate groups is particularly referred to as an "S-oligonucleotide". All the oligonucleotides listed in Table 1 were S-oligonucleotides.

### Example 3: Analysis (1) of REST Expression Levels Using Gapmer-Type ASOs

Out of the gapmer-type ASOs prepared in Example 2, those listed in Table 1 were used to analyze the REST expression levels as follows.

Human lung cancer cells (A549 cells) were seeded on a 24-well plate such that the number of the cells was 2.0×10⁴ cells, and were cultured at 37°C at 5% CO₂ for 24 hours. Then, lipofection was conducted using Lipofectamine 3000 (Invitrogen) with the final concentration of each ASO being 100 nM. After cultured for another 24 hours, the cells were collected. Then, RNA was extracted using RNeasy Plus Micro Kit (QIAGEN), and reverse transcription was conducted using SuperScript IV VILO Master Mix. Step One Plus Real Time PCR System was used to conduct RT-PCR using TaqMan Fast Advanced Master Mix (Applied Biosystems) and TaqMan gene expression assay (manufactured by Applied Biosystems). The primers and probes used were Hs05028212_s1 (manufactured by Thermo Fisher Scientific) for REST and Hs99999903_m1 (manufactured by Thermo Fisher Scientific) for actin.

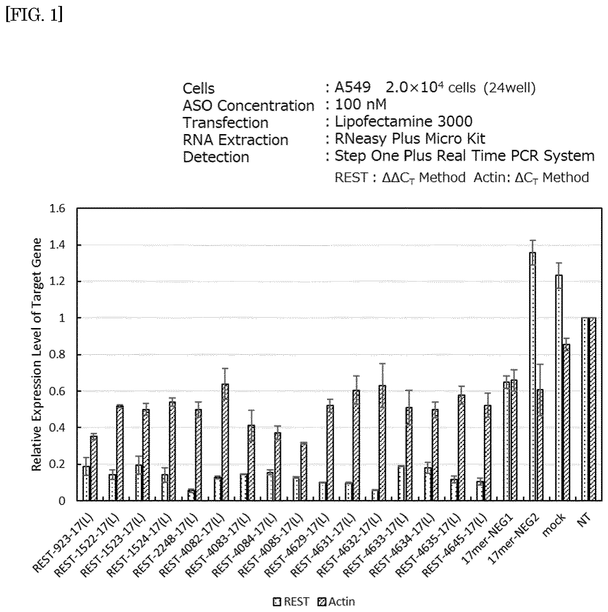

Sterile water was used for control (mock). An untreated sample was indicated as control (NT) (the same applies hereinafter). The results are shown in FIG. 1.

As shown in FIG. 1, it can be seen that all the gapmer-type ASOs listed in Table 2 suppressed the expression of the REST.

Since the concentration as high as 100 nM was employed, it was difficult to confirm the difference in activity in detail. Therefore, REST-4632-17(L) (SEQ ID NO: 30) and NEG2, with which a decrease in the actin level was relatively small and off-target was less likely to occur, were used to optimize the concentration.

### Example 4: Optimization of ASO Concentration Using REST-4632-17(L) and NEG2

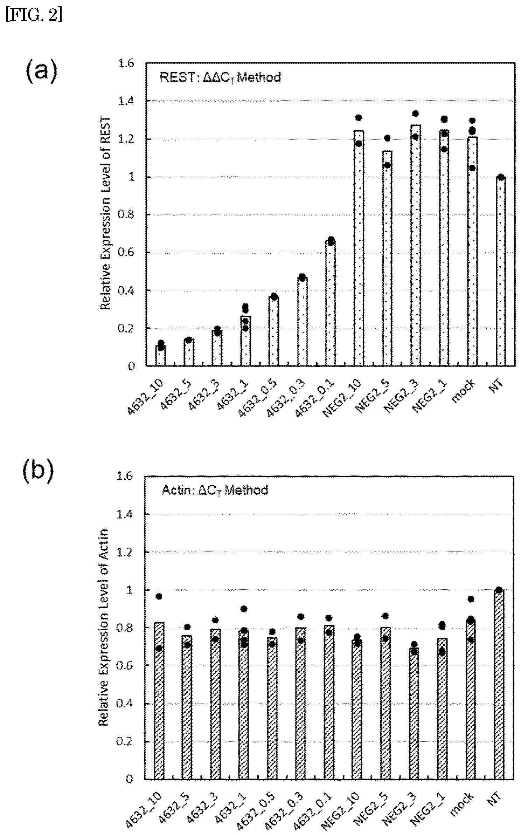

Human lung cancer cells (A549 cells) were seeded on a 24-well plate such that the number of the cells was 2.0×10⁴ cells, and were cultured at 37°C at 5% CO₂ for 24 hours. Then, lipofection was conducted using Lipofectamine 3000 (Invitrogen) with the final concentration of each ASO (REST-4632-17(L) (SEQ ID NO: 30) or NEG2) being 10 nM, 5 nM, 3 nM, 1 nM, 0.5 nM, 0.3 nM, or 0.1 nM (these cases will be abbreviated as 4632_10, 4632_5, 4632_3, 4632_1, 4632_0.5, 4632_0.3, 4632_0.1, NEG2_10, NEG2_5, NEG2_3, NEG_1 hereinafter). After cultured for another 24 hours, the cells were collected. Then, RNA was extracted using RNeasy Plus Micro Kit (QIAGEN), and reverse transcription was conducted using SuperScript IV VILO Master Mix. Step One Plus Real Time PCR System was used to conduct RT-PCR using TaqMan Fast Advanced Master Mix (Applied Biosystems) and TaqMan gene expression assay (Applied Biosystems). The primers and probes used were Hs05028212_s1 for REST and Hs99999903_m1 for actin. Sterile water was used for control (mock). An untreated sample was indicated as control (NT). The results are shown in FIG. 2.

As shown in FIG. 2(a), it can be seen that when REST-4632-17(L) (SEQ ID NO: 30) was used, the expression of the REST was suppressed in a concentration-dependent manner, but the expression of actin was not affected (FIG. 2(b)).

### Example 5: Analysis (2) of REST Expression Levels Using Gapmer-Type ASOs

Out of the gapmer-type ASOs prepared in Example 2, those listed in Table 2 were used to analyze the REST expression levels as follows.

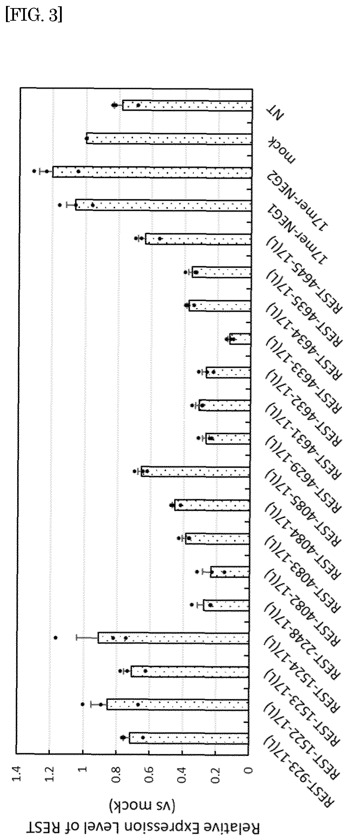

Human lung cancer cells (A549 cells) were seeded on a 24-well plate such that the number of the cells was 2.0×10⁴ cells, and were cultured at 37°C at 5% CO₂ for 24 hours. Then, the REST expression levels were analyzed using the gapmer-type ASOs in the same manner as in Example 3, except that lipofection was conducted using Lipofectamine 3000 (Invitrogen) with the final concentration of each ASO being 0.5 nM. The results are shown in FIG. 3.

As shown in FIG. 3, it can be seen that in particular, REST-2248-17(L) (SEQ ID NO: 22), REST-4082-17(L) (SEQ ID NO: 23), and REST-4633-17(L) (SEQ ID NO: 30) out of the gapmer-type ASOs listed in Table 2 more effectively suppressed the expression of the REST.

### Example 6: Evaluation of Concentration-Dependent Activity of Gapmer-Type ASOs

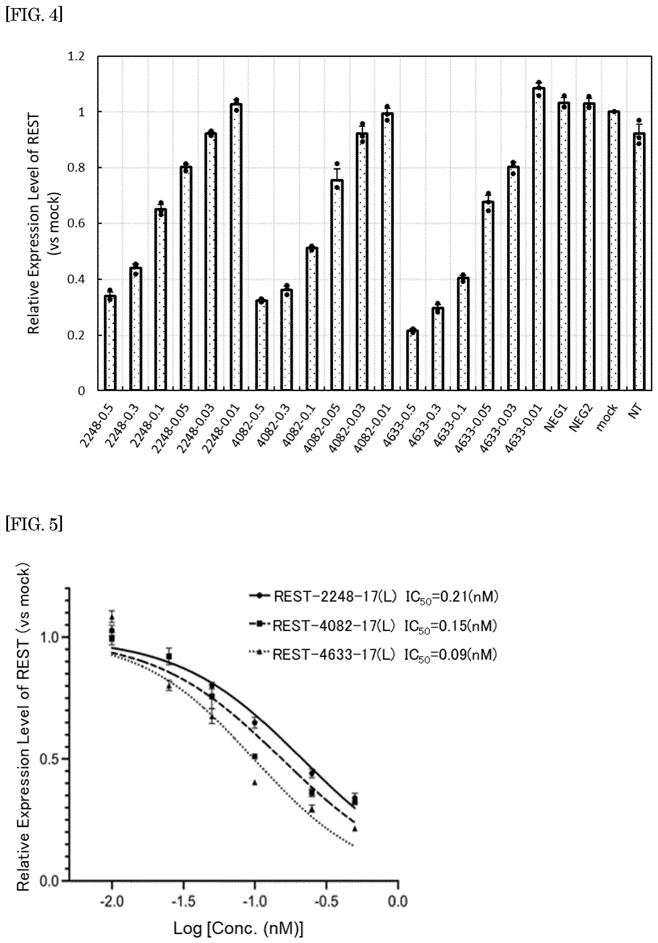

Human lung cancer cells (A549 cells) were seeded on a 24-well plate such that the number of the cells was 2.0×10⁴ cells, and were cultured at 37°C at 5% CO₂ for 24 hours. Then, lipofection was conducted using Lipofectamine 3000 (Invitrogen) with the final concentration of each ASO (REST-2248-17(L) (SEQ ID NO: 22), REST-4082-17(L) (SEQ ID NO: 23), or REST-4633-17(L) (SEQ ID NO: 30)) being 0.5 nM, 0.3 nM, 0.1 nM, 0.05 nM, 0.03 nM, or 0.01 nM (these cases will be abbreviated as 2248_0.5, 2248_0.3, 2248_0.1, 2248_0.05, 2248_0.03, 2248_0.01, 4082_0.5, 4082_0.3, 4082_0.1, 4082_0.05, 4082_0.03, 4082_0.01, 4633_0.5, 4633_0.3, 4633_0.1, 4633_0.05, 4633_0.03, and 4633_0.01 hereinafter). After cultured for another 24 hours, the cells were collected. Then, RNA was extracted using RNeasy Plus Micro Kit (QIAGEN), and reverse transcription was conducted using SuperScript IV VILO Master Mix (Invitrogen). Step One Plus Real Time PCR System was used to conduct RT-PCR using TaqMan Fast Advanced Master Mix (Applied Biosystems) and TaqMan gene expression assay (Applied Biosystems). The primers and probes used were Hs05028212_s1 for REST and Hs99999903_m1 for actin. Sterile water was used for control (mock). An untreated sample was indicated as control (NT). The results are shown in FIG. 4. The results of the relative REST expression levels versus the logarithmic values of the ASO concentrations are shown in FIG. 5.

As shown in FIG. 4, all the gapmer-type ASOs (REST-2248-17(L) (SEQ ID NO: 22), REST-4082-17(L) (SEQ ID NO: 23), and REST-4633-17(L) (SEQ ID NO: 30)) used increased the relative REST expression levels in a concentration-dependent manner. Also, as shown in FIG. 5, the IC₅₀ values of these ASOs were very small, and it can be seen that the activity thereof to suppress the expression of the REST was high.

### Example 7: Analysis of Suppression of Cell Proliferation of A549 Cells

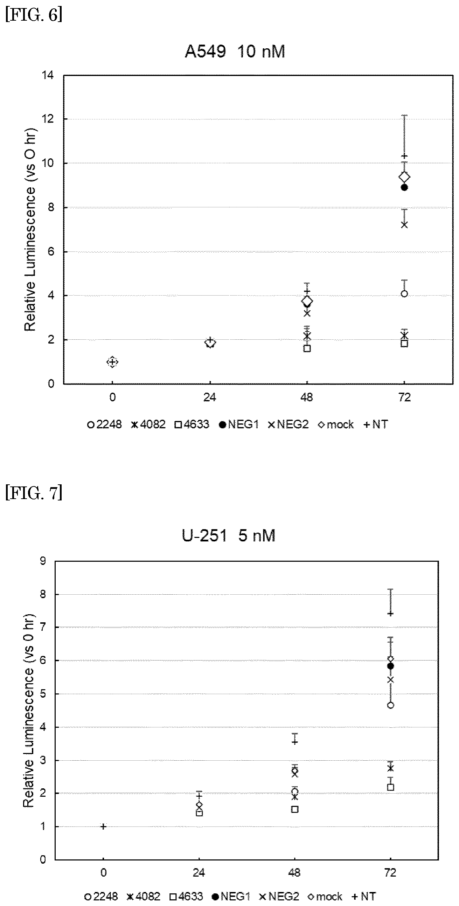

Human lung cancer cells (A549 cells) were seeded on a 96-well plate such that the number of the cells was 1.0×10³ cells, and were cultured at 37°C at 5% CO₂ for 24 hours. Then, lipofection was conducted using Lipofectamine 3000 (Invitrogen) with the final concentration of each ASO (REST-2248-17(L) (SEQ ID NO: 22), REST-4082-17(L) (SEQ ID NO: 23), or REST-4633-17(L) (SEQ ID NO: 30)) being 10 nM (these cases will be abbreviated as 2248, 4082, and 4633 hereinafter). The time point when the lipofection had been conducted was taken as 0 hour, and the luminescence of viable cells was measured using CellTiter-Glo 3D Cell Viability Assay (Promega) every 24 hours to calculate the cell proliferation ratio based on a value relative to the luminescence level at 0 hour. The results are shown in FIG. 6.

As shown in FIG. 6, it can be seen that the cell proliferation of the A549 cells was suppressed due to the expression of the REST being suppressed using REST-2248-17(L) (SEQ ID NO: 22), REST-4082-17(L) (SEQ ID NO: 23), or REST-4633-17(L) (SEQ ID NO: 30), and the ability thereof to suppress cell proliferation was proportional to the ability to suppress the expression of the REST (2248 < 4082 < 4633).

### Example 8: Analysis of Suppression of Cell Proliferation of U-251 Cells

Human glioblastoma cells (U-251 cells) were seeded on a 96-well plate such that the number of the cells was 5.0×10² cells, and were cultured at 37°C at 5% CO₂ for 24 hours. Then, lipofection was conducted using Lipofectamine 3000 (Invitrogen) with the final concentration of each ASO (REST-2248-17(L) (SEQ ID NO: 22), REST-4082-17(L) (SEQ ID NO: 23), or REST-4633-17(L) (SEQ ID NO: 30)) being 5 nM (these cases will be abbreviated as 2248, 4082, and 4633 hereinafter). The time point when the lipofection had been conducted was taken as 0 hour, and the luminescence of viable cells was measured using CellTiter-Glo 3D Cell Viability Assay (Promega) every 24 hours to calculate the cell proliferation ratio based on a value relative to the luminescence level at 0 hour. The results are shown in FIG. 7.

As shown in FIG. 7, it can be seen that the cell proliferation of the U-251 cells was suppressed due to the expression of the REST being suppressed using REST-2248-17(L) (SEQ ID NO: 22), REST-4082-17(L) (SEQ ID NO: 23), or REST-4633-17(L) (SEQ ID NO: 30), and the ability thereof to suppress cell proliferation was proportional to the ability to suppress the expression of the REST (2248 < 4082 < 4633).

### Example 9: Activity to Suppress Cell Proliferation of A549 Cells

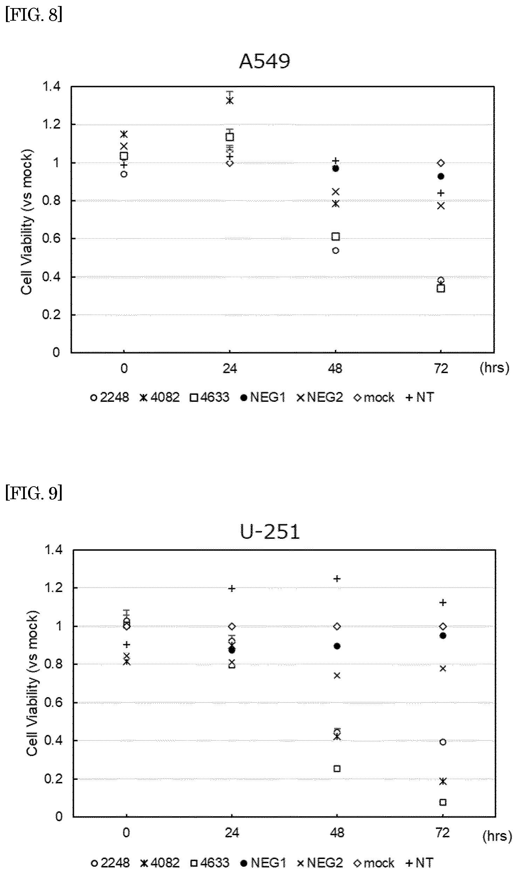

Based on the publication levels of the viable cells relative to the luminescence levels of the mock at various time points (0 hour, 24 hours, 48 hours, and 72 hours) obtained in the analyses of suppression of cell proliferation of human lung cancer cells (A549 cells) conducted in Example 7, the cell proliferation ratios were calculated. The results are shown in FIG. 8.

As shown in FIG. 8, it can be seen that the cell proliferation of the A549 cells was suppressed due to the expression of the REST being suppressed using REST-2248-17(L) (SEQ ID NO: 22), REST-4082-17(L) (SEQ ID NO: 23), or REST-4633-17(L) (SEQ ID NO: 30).

### Example 10: Activity to Suppress Cell Proliferation of U-251 Cells

Based on the luminescence levels of the viable cells relative to the luminescence levels of the mock at various time points (0 hour, 24 hours, 48 hours, and 72 hours) obtained in the analyses of suppression of cell proliferation of human glioblastoma cells (U-251 cells) conducted in Example 8, the cell proliferation ratios were calculated. The results are shown in FIG. 9.

As shown in FIG. 9, it can be seen that the cell proliferation of the U-251 cells was suppressed due to the expression of the REST being suppressed using REST-2248-17(L) (SEQ ID NO: 22), REST-4082-17(L) (SEQ ID NO: 23), or REST-4633-17(L) (SEQ ID NO: 30).

### Example 11: Analysis of Expression of REST Protein Using Anti-REST Antibody

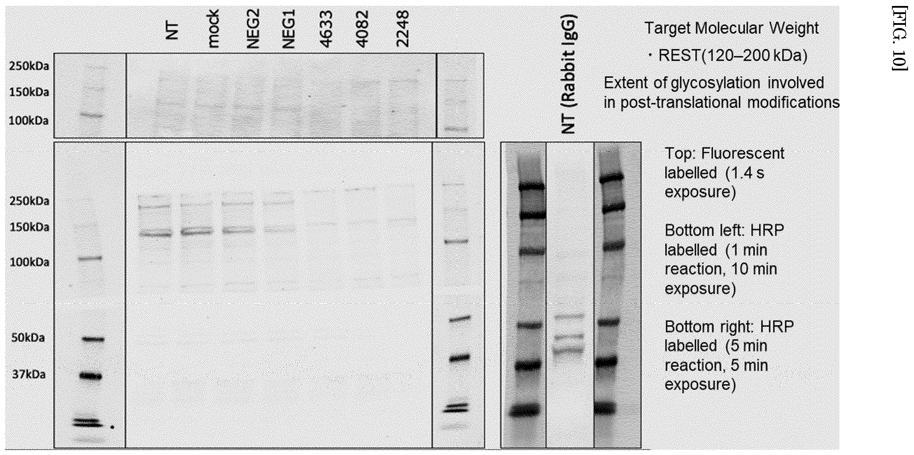

Human lung cancer cells (A549 cells) were seeded on a 6-well plate such that the number of the cells was 9.0×10⁴ cells, and were cultured at 37°C at 5% CO₂ for 24 hours. Then, lipofection was conducted using Lipofectamine 3000 (Invitrogen) with the final concentration of each ASO (REST-2248-17(L) (SEQ ID NO: 22), REST-4082-17(L) (SEQ ID NO: 23), REST-4633-17(L) (SEQ ID NO: 30), NEG1, NEG2, mock, and NT) being 5 nM (these cases will be abbreviated as 2248, 4082, 4633, NEG1, NEG2, mock and NT hereinafter). After cultured for another 48 hours, the cells were collected. Then, the cells were homogenized in the RIPA buffer, and protein assay was conducted using Pierce BCA Protein Assay Kit (Thermo Scientific). Subsequently, equal amounts of proteins were thermally denatured in Laemmli Sample Buffer (BIO-RAD) at 95°C for 5 minutes. Then, SDS-PAGE was conducted, and the gel was transferred to a membrane. Thereafter, antibody reaction was conducted in accordance with the protocol of iBind Flex Western Device (Invitrogen). Note that the antibodies used were REST/NRSF (Rabbit pAb) (07-579, Merck Millipore) and β-Actin (mouse mAb) (3700, Cell Signaling). The results are shown in FIG. 10.

### Example 12: Analysis (3) of REST Expression Levels Using Gapmer-Type ASOs (REST Expression Levels Depending on Difference in Modified Nucleic Acid)

Out of the gapmer-type ASOs prepared in Example 2, REST-2248-17(L) (SEQ ID NO: 22), REST-4082-17(L) (SEQ ID NO: 23), and REST-4633-17(L) (SEQ ID NO: 30) listed in Table 1, which included 2',4'-BNA/LNA, and REST-2248-17(Y) (SEQ ID NO: 34), REST-4082-17(Y) (SEQ ID NO: 35), and REST-4633-17(Y) (SEQ ID NO: 36) listed in Table 2, which included amide BNA (AmNA), were used, and the REST expression levels were compared as follows.

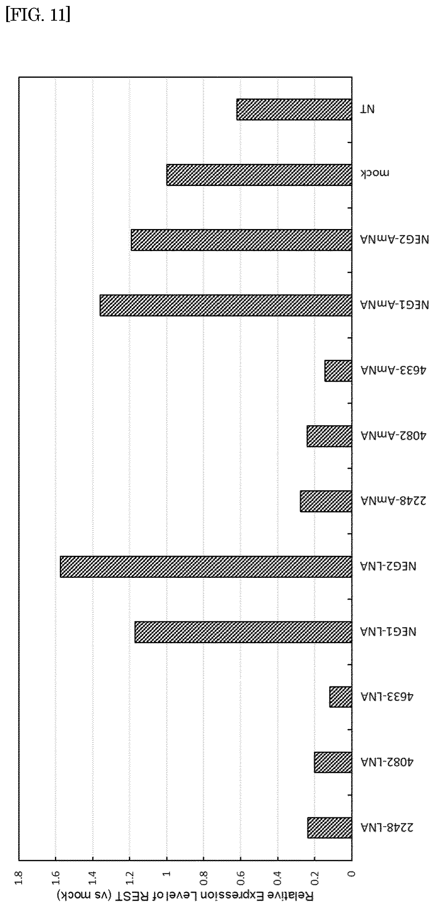

Human lung cancer cells (A549 cells) were seeded on a 24-well plate such that the number of the cells was 2.0×10⁴ cells, and were cultured at 37°C at 5% CO₂ for 24 hours. Then, lipofection was conducted with the final concentration of each ASO being 1 nM (these cases will be abbreviated as 2248-LNA, 4082-LNA, 4633-LNA, NEG1-LNA, NEG2-LNA, 2248-AmNA, 4082-AmNA, 4633-AmNA, NEG1-AmNA, NEG2-AmNA mock, and NT hereinafter). After cultured for another 24 hours, the cells were collected. Then, RNA was extracted using RNeasy Plus Micro Kit (QIAGEN), and reverse transcription was conducted using SuperScript IV VILO Master Mix (Invitrogen). Step One Plus Real Time PCR System was used to conduct RT- PCR using TaqMan Fast Advanced Master Mix (Applied Biosystems) and TaqMan gene expression assay (Applied Biosystems). The primers and probes used were Hs05028212_s1 for REST and Hs99999903_m1 for actin. The results are shown in FIG. 11.

As shown in FIG. 11, it can be seen that the expression of the REST could be effectively suppressed in both cases where LNA (i.e., 2',4'-BNA/LNA) was introduced into the ASO and AmNA (amide BNA) was introduced into the ASO.

### Industrial Applicability

The present invention is useful for, for example, development of therapeutic agents for various nervous system diseases (e.g., malignant tumors, dementia, and central nervous system diseases), diabetes, and the like, which are caused by the expression of the REST

## Claims

1. An oligonucleotide or pharmacologically acceptable salt thereof capable of regulating expression of REST, comprising a nucleotide sequence complementary to a continuous sequence with at least 15 bases in a target region included in a base sequence of SEQ ID NO: 1.

2. The oligonucleotide or pharmacologically acceptable salt thereof according to claim 1, wherein the oligonucleotide has 16 to 25 bases.

3. The oligonucleotide or pharmacologically acceptable salt thereof according to claim 1, wherein the oligonucleotide includes a sequence complementary to a target region having a sequence within a base sequence between positions 900 to 4700 of SEQ ID NO: 1.

4. The oligonucleotide or pharmacologically acceptable salt thereof according to claim 1, wherein a 5' terminus of the target region corresponds to position 923, 1522, 1523, 1524, 2248, 4082, 4083, 4084, 4085, 4629, 4631, 4632, 4633, 4634, 4635, or 4645 of SEQ ID NO: 1.

5. The oligonucleotide or pharmacologically acceptable salt thereof according to claim 1, wherein the oligonucleotide is
REST-923-17(L) (SEQ ID NO: 18),
REST-1522-17(L) (SEQ ID NO: 19),
REST-1523-17(L) (SEQ ID NO: 20),
REST-1524-17(L) (SEQ ID NO: 21),
REST-2248-17(L) (SEQ ID NO: 22),
REST-4082-17(L) (SEQ ID NO: 23),
REST-4083-17(L) (SEQ ID NO: 24),
REST-4084-17(L) (SEQ ID NO: 25),
REST-4085-17(L) (SEQ ID NO: 26),
REST-4629-17(L) (SEQ ID NO: 27),
REST-4631-17(L) (SEQ ID NO: 28),
REST-4632-17(L) (SEQ ID NO: 29),
REST-4633-17(L) (SEQ ID NO: 30),
REST-4634-17(L) (SEQ ID NO: 31),
REST-4635-17(L) (SEQ ID NO: 32),
REST-4645-17(L) (SEQ ID NO: 33),
REST-2248-17(Y) (SEQ ID NO: 34),
REST-4082-17(Y) (SEQ ID NO: 35), or
REST-4633-17(Y) (SEQ ID NO: 36).

6. The oligonucleotide or pharmacologically acceptable salt thereof according to claim 1, comprising at least one nucleoside structure represented by Formula (I) below: 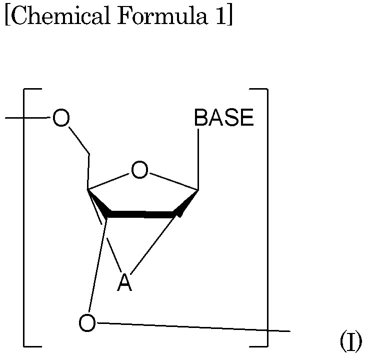
(in Formula (I),
BASE represents a purin-9-yl group that may have any one or more substituents selected from an a group, or a 2-oxo-1,2-dihydropyrimidin-1-yl group that may have any one or more substituents selected from the α group, the a group consisting of a hydroxy group, a hydroxy group protected by a protecting group for nucleic acid synthesis, linear alkyl groups having 1 to 6 carbon atoms, linear alkoxy groups having 1 to 6 carbon atoms, a mercapto group, a mercapto group protected by a protecting group for nucleic acid synthesis, linear alkylthio groups having 1 to 6 carbon atoms, an amino group, linear alkylamino groups having 1 to 6 carbon atoms, an amino group protected by a protecting group for nucleic acid synthesis, and halogen atoms, and
A is a divalent group represented by: 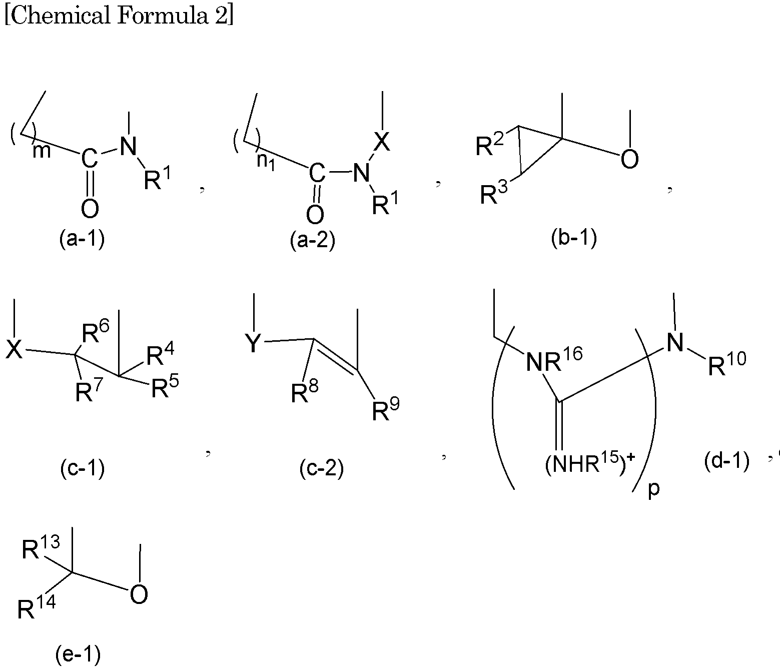 or
where
R¹ represents a hydrogen atom, an alkyl group having 1 to 7 carbon atoms that may be branched or cyclic, an alkenyl group having 2 to 7 carbon atoms that may be branched or cyclic, an aryl group having 3 to 12 carbon atoms that may have any one or more substituents selected from the a group and may have a hetero atom, an aralkyl group with an aryl moiety having 3 to 12 carbon atoms that may have any one or more substituents selected from the a group and may have a hetero atom, or an amino group protecting group for nucleic acid synthesis;
R² and R³ are independently a hydrogen atom, an alkyl group having 1 to 7 carbon atoms that may have undergone substitution with an aryl group having 3 to 12 carbon atoms that may have a hetero atom, and may be branched or cyclic, or an aralkyl group with an aryl moiety having 3 to 12 carbon atoms that may have a hetero atom, or
R² and R³ together represent -(CH2)_{q}- (where q is an integer from 2 to 5);
R⁴ and R⁵ are independently a group selected from the group consisting of a hydrogen atom, a hydroxy group, alkyl groups having 1 to 7 carbon atoms that may be branched or cyclic, alkoxy groups having 1 to 7 carbon atoms that may be branched or cyclic, an amino group, and an amino group protected by a protecting group for nucleic acid synthesis, or R⁴ and R⁵ together form =C(R¹¹)R¹² (where R¹¹ and R¹² independently represent a hydrogen atom, a hydroxy group, a hydroxy group protected by a protecting group for nucleic acid synthesis, a mercapto group, a mercapto group protected by a protecting group for nucleic acid synthesis, an amino group, a linear or branched alkoxy group having 1 to 6 carbon atoms, a linear or branched alkylthio group having 1 to 6 carbon atoms, a cyanoalkoxy group having 1 to 6 carbon atoms, or a linear or branched alkylamino group having 1 to 6 carbon atoms);
R⁶ and R⁷ are independently a hydrogen atom, an alkyl group having 1 to 7 carbon atoms that may be branched or cyclic, an alkoxy group having 1 to 7 carbon atoms that may be branched or cyclic, or a linear or branched alkylthio group having 1 to 6 carbon atoms;
R⁸ represents a hydrogen atom, an alkyl group having 1 to 7 carbon atoms that may be branched or cyclic, an alkoxy group having 1 to 7 carbon atoms that may be branched or cyclic, or a linear or branched alkylthio group having 1 to 6 carbon atoms;
R⁹ is a hydrogen atom, a hydroxy group, an alkyl group having 1 to 7 carbon atoms that may be branched or cyclic, an alkoxy group having 1 to 7 carbon atoms that may be branched or cyclic, an amino group, or an amino group protected by a protecting group for nucleic acid synthesis;
R¹⁰ is a hydrogen atom, an alkyl group having 1 to 7 carbon atoms that may be branched or cyclic, an amino group protecting group, or 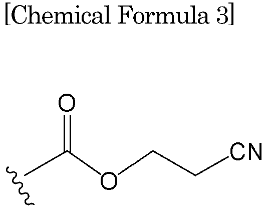
or -(C=(NHR¹⁷)⁺)-NR¹⁸R¹⁹ (where R¹⁷, R¹⁸, and R¹⁹ are independently a hydrogen atom, an alkyl group having 1 to 7 carbon atoms that may be branched or cyclic, an amino group protecting group, or 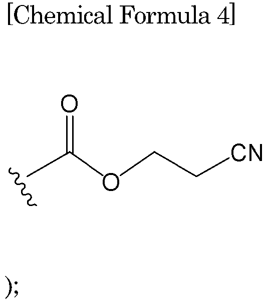
R¹³ and R¹⁴ are independently a group selected from the group consisting of a hydrogen atom, a hydroxy group, alkyl groups having 1 to 7 carbon atoms that may be branched or cyclic, alkoxy groups having 1 to 7 carbon atoms that may be branched or cyclic, an amino group, and an amino group protected by a protecting group for nucleic acid synthesis;
m is an integer from 0 to 2;
n is an integer of 0 or 1;
when R¹⁰ is a hydrogen atom, an alkyl group having 1 to 7 carbon atoms that may be branched or cyclic, an amino group protecting group, or 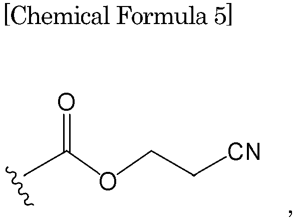
p is 1, and R¹⁵ and R¹⁶ are independently a hydrogen atom, an alkyl group having 1 to 7 carbon atoms that may be branched or cyclic, an amino group protecting group, or 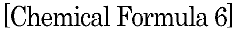 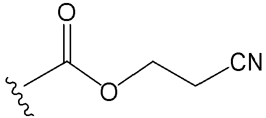 or
when R¹⁰ is -(C=(NHR¹⁷)⁺)-NR¹⁸R¹⁹, p is 0;
X is an oxygen atom, a sulfur atom, or an amino group; and
Y is an oxygen atom or a sulfur atom).

7. The oligonucleotide or pharmacologically acceptable salt thereof according to claim 6,
wherein the oligonucleotide is a gapmer that includes a gap region having 9 to 15 bases, a 5' wing having 3 to 5 bases, and 3' wing having 3 to 5 bases,
the gap region is located between the 5' wing and the 3' wing, and
the 5' wing and the 3' wing include at least one nucleoside structure represented by Formula (I).

8. The oligonucleotide or pharmacologically acceptable salt thereof according to claim 1, wherein bonds between nucleotides included in the oligonucleotide include phosphorothioate.

9. A REST expression suppressing agent comprising the oligonucleotide or pharmacologically acceptable salt thereof according to any one of claims 1 to 8.

10. A pharmaceutical composition comprising the oligonucleotide or pharmacologically acceptable salt thereof according to any one of claims 1 to 8.

11. The pharmaceutical composition according to claim 10 for use to treat a nervous system disease.
